# EUROPEAN PATENT SPECIFICATION

(11) **EP 1 109 901 B1**
(45) Date of publication and mention of the grant of the patent: **25.10.2006**
(21) Application number: 99943118.2
(22) Date of filing: 03.09.1999
(51) Int. Cl.: C12N 15/12, A61K 49/00, C07K 14/705, C07K 16/28, A61P 25/00, C12N 5/10

(54) **GABA B RECEPTOR SUBTYPES GABA B-R1c AND GABA B-R2 AND HETERODIMERS THEREOF**
GABA B-REZEPTOR-SUBTYPEN GABA B-R1c UND GABA B-R2 UND DEREN HETERODIMERE
SOUS-TYPES GABA B-R1C ET GABA B-R2 DU RECEPTEUR GABA B ET LEURS HETERODIMERES

(30) Priority: 07.09.1998 GB 9819420; 09.10.1998 US 103670 P
(43) Date of publication of application: 27.06.2001
(73) Proprietor: GLAXO GROUP LIMITED, Greenford, Middlesex UB6 ONN (GB)
(72) Inventor: BARNES, Ashley, Antony, Stevenage, Hertfordshire SG1 2NY (GB); WISE, Alan, Stevenage, Hertfordshire SG1 2NY (GB); MARSHALL, Fiona, Hamilton, Stevenage, Hertfordshire SG1 2NY (GB); FRASER, Neil, James, Stevenage, Hertfordshire SG1 2NY (GB); WHITE, Julia, Helen, Margaret, Stevenage, Hertfordshire SG1 2NY (GB); FOORD, Steven, Michael, Stevenage, Hertfordshire SG1 2NY (GB)
(74) Representative: Lawrence, Geoffrey Mark Prouse
(86) International application number: PCT/GB1999/002918
(87) International publication number: WO 2000/014222

(56) References cited:
- EP-A- 0 937 777
- WO-A-00/00602
- WO-A-97/46675
- WO-A-99/20751
- WO-A-99/21890
- WO-A-99/40114
- WO-A-99/42603
- WO-A-99/51636
- WO-A-99/51641
- KAUPMANN K ET AL: "EXPRESSION CLONING OF GABAB RECEPTORS UNCOVERS SIMILARITY TO METABOTROPIC GLUTAMATE RECEPTORS" NATURE,GB,MACMILLAN JOURNALS LTD. LONDON, vol. 386, no. 6622, 20 March 1997 (1997-03-20), page 239-246 XP002032306 ISSN: 0028-0836 cited in the application
- EST EMBASE AC H14151, Sequence characterization ym62d04.r1, Homo sapiens cDNA clone 163495 5'. 12-07-1995 XP002127119 cited in the application
- EST EMBASE AC R76089 sequence characterization yi93c05.s1, Homo sapiens cDNA clone 144661 3'. 10-06-1995 XP002127120 cited in the application
- EST EMBASE AC R80651 Sequence characterization yi93c05.s1, Homo sapiens cDNA clone 146792 3'. 11-06-1995 XP002127121 cited in the application
- EST EMBASE AC AA324303, Sequence characterization EST27126, Cerebellum II Homo sapiens cDNA 5' end. 18-04-1997 XP002127122 cited in the application
- EST EMBASE AC T07621, Sequence characterization EST05511, Homo sapiens cDNA clone HFBEL81. 25-07-1995 XP002127123 cited in the application
- EST EMBASE AC Z43654, Sequence characterization Homo sapiens partial cDNA sequence; clone c-1hh04 21-09-1995 XP002127124 cited in the application
- JONES ET AL: "GABAB receptors function as heteromeric assembly of the subunits GABABR1 and GABABR2" NATURE,GB,MACMILLAN JOURNALS LTD. LONDON, vol. 396, no. 396, 17 December 1998 (1998-12-17), page 674-679 XP002116148 ISSN: 0028-0836
- WHITE J H ET AL: "Heterodimerization is required for the formation of a functional GABAB receptor" NATURE,GB,MACMILLAN JOURNALS LTD. LONDON, vol. 396, no. 396, 17 December 1998 (1998-12-17), page 679-682 XP002116149 ISSN: 0028-0836
- BRAUNER-OSBORNE H, KROGSGAARD-LARSEN P. : "Functional pharmacology of cloned heterodimeric GABAB receptors expressed in mammalian cells." BR J PHARMACOL. , vol. 127, no. 7, December 1999 (1999-12), pages 1370-1374, XP000884864
- PFAFF T., MALITSCHEK B., KAUPMANN K., BETTLER B., KARSCHIN K.;: "Alternative splicing generates a novel isoform of the rat metabotropic GABA(B)R1 receptor." EUR J NEUROSCI., vol. 11, no. 8, August 1999 (1999-08), pages 2874-2882, XP000884866
- ISOMOTO S, KAIBARA M, SAKURAI-YAMASHITA Y, NAGAYAMA Y, UEZONO Y, YANO K, TANIYAMA K : "Cloning and tissue distribution of novel splice variants of the rat GABAB receptor. " BIOCHEM BIOPHYS RES COMMUN , vol. 253, no. 1, December 1998 (1998-12), pages 10-15, XP000877283
- WISE, A. ET AL.: "Calcium sensing properties of the GABAB receptor." NEUROPHARMACOLOGY., vol. 38, no. 11, November 1999 (1999-11), pages 1647-1656, XP000865720 PERGAMON PRESS, OXFORD., GB ISSN: 0028-3908

## Description

### Field of the Invention

The present invention relates to the novel GABA_{B} receptor subtypes GABA_{B}-R1c and GABA_{B}-R2 as well as to a novel, functional GABA_{B} receptor which comprises a heterodimer of GABA_{B}-R1 and GABA_{B}-R2 receptor subunits. The present invention also relates to variants of the receptors, nucleotide sequences encoding the receptors and variants thereof and novel vectors, stable cell lines, antibodies, screening methods, methods of treatment and methods of receptor production.

### Background of the Invention

GABA (γ-amino-butyric acid) is the main inhibitory neurotransmitter in the central nervous system (CNS) activating two distinct families of receptors; the ionotropic GABA_{A} and GABA_{C} receptors for fast synaptic transmissions, and the metabotropic GABA_{B} receptors governing a slower synaptic transmission. GABA_{B} receptors are members of the superfamily of 7-transmembrane G protein-coupled receptors. Activation results in signal transduction through a variety of pathways mediated principally via members of the Gᵢ/Gₒ family of pertussis toxin-sensitive G proteins. GABA_{B} receptors have been shown to inhibit N, P/Q and T-type Ca²⁺ channels in a pertussis toxin-sensitive manner (Kobrinsky *et al.,* 1993; Menon-Johansson *et al.,* 1993; Harayama *et al.,* 1998) and indeed there is also some evidence for direct interactions between GABA_{B} receptors and Ca²⁺ channels since Ca²⁺ channel ligands can modify the binding of GABA_{B} agonists (Ohmori *et al.,* 1990). GABA_{B} receptor-mediated Ca²⁺ channel inhibition is the principle mechanism for presynaptic inhibition of neurotransmitter release. Post-synaptically the major effect of GABA_{B} receptor activation is to open potassium channels, to generate post-synaptic inhibitory potentials.

Autoradiographic studies show that GABA_{B} receptors are abundant and heterogeneously distributed throughout the CNS, with particularly high levels in the molecular layer of the cerebellum, interpeduncular nucleus, frontal cortex, olfactory nuclei and thalamic nuclei. GABA_{B} receptors are also widespread in the globus pallidus, temporal cortex, raphe magnus and spinal cord (Bowery *et al.,* 1987). GABA_{B} receptors are an important therapeutic target in the CNS for conditions such as spasticity, epilepsy, Alzheimer's disease, pain, affective disorders and feeding. GABA_{B} receptors are also present in the peripheral nervous system, both on sensory nerves and on parasympathetic nerves. Their ability to modulate these nerves gives them potential as targets in disorders of the lung, Gl tract and bladder (Kerr and Ong, 1995; 1996; Malcangio and Bowery, 1995).

Despite the widespread abundance of GABA_{B} receptors, considerable evidence from neurochemical, electrophysiological and behavioural studies suggests that multiple subtypes of GABA_{B} receptors exist. This heterogeneity of GABA_{B} receptors may allow the development of selective ligands, able to target specific aspects of GABA_{B} receptor function. This would lead to the development of drugs with improved selectivity profiles relative to current compounds (such as bactofen) which are relatively non-selective and show a variety of undesirable behavioural actions such as sedation and respiratory depression. Multiple receptor subtypes are best classified by the differing profiles of agonist and antagonist ligands.

To date screening for GABA_{B} ligands and subsequent structure / activity determinations has relied on radioligand binding assays to rat brain membranes. Further analysis of such ligands in animal models has indicated differences in their behavioural profile. However, due to the absence of cloned GABA_{B} receptors the molecular basis for such differences has not been defined, and therefore it has not been possible to optimise GABA_{B} ligands for therapeutic use.

GABA_{B} receptors were first described nearly 20 years ago (Hill and Bowery, 1981), but despite extensive efforts using conventional expression cloning strategies, for example in *Xenopus* oocytes, or cloning based on sequence homology, the molecular nature of the GABA_{B} receptor remained elusive. The development of a high affinity antagonist for the receptor finally allowed Kaupmann *et al.,* (1997) to expression clone the receptor from a rat cerebral cortex cDNA using a radioligand binding assay. Two splice variants of the receptor were identified, GABA_{B}-R1a encoding a 960 amino acid protein and GABA_{B}-R1b, encoding an 844 amino acid protein, differing only in the lengths of their N-termini. These two splice variants have distinct spatial distributions within the brain, but both reside within neuronal rather than glial cells. Pharmacologically, the two splice variants are similar, showing binding affinities for a range of antagonists, but about 10 fold lower than those of native receptors, as well as agonist displacement constants which are about 100-150 fold lower than those of native receptors. These observations have led to speculation that the cloned receptor was a low affinity receptor and an additional high affinity, pharmacologically distinct GABA_{B} receptor subtype could exist in the brain. Alternatively, it was argued that G-protein coupling was inefficient or the receptor was desensitising in the recombinant systems used.

A number of groups working in the area have, however, found that the cloned receptor fails to behave as a functional GABA_{B} receptor either in mammalian cells or in *Xenopus* oocytes. The present invention describes the cloning of a novel human GABA_{B} receptor subtype, GABA_{B}-R2, the identification of a novel splice variant GABA_{B}-R1c, and the surprising observation that GABA_{B}-R1 and GABA_{B}-R2 strongly interact via their C-termini to form heterodimers. Co-expression of GABA_{B-}R1 and GABA_{B}-R2 allows trafficking of GABA_{B}-R1 to the cell surface and results in a high affinity functional GABA_{B} receptor in both mammalian cells and *Xenopus* oocytes.

These surprising findings provide a unique opportunity to define GABA_{B} subtypes at the molecular level, which in turn will lead to the identification of novel subtype-specific drugs.

### Summary of the Invention

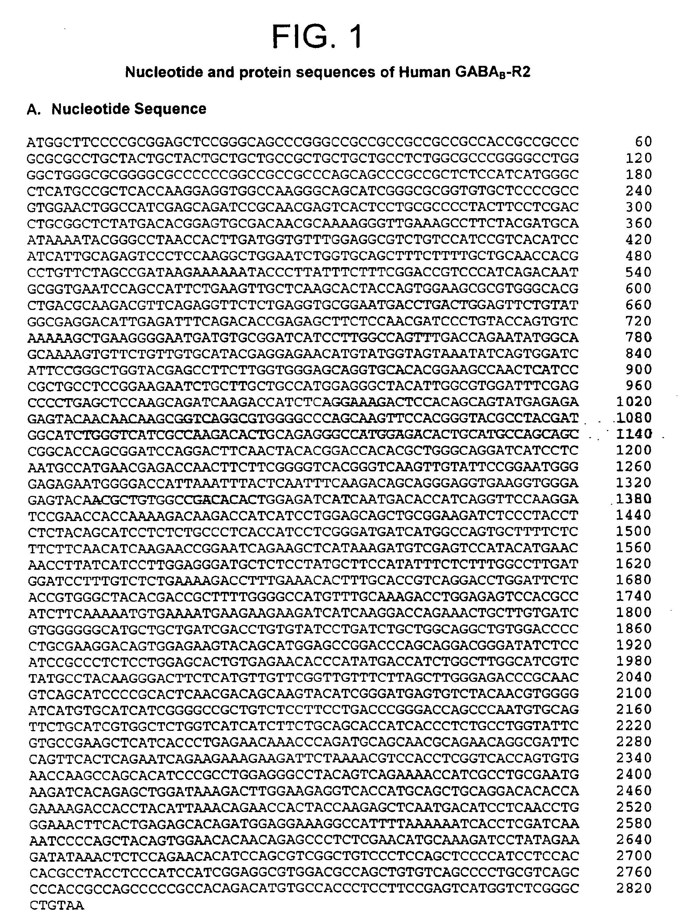

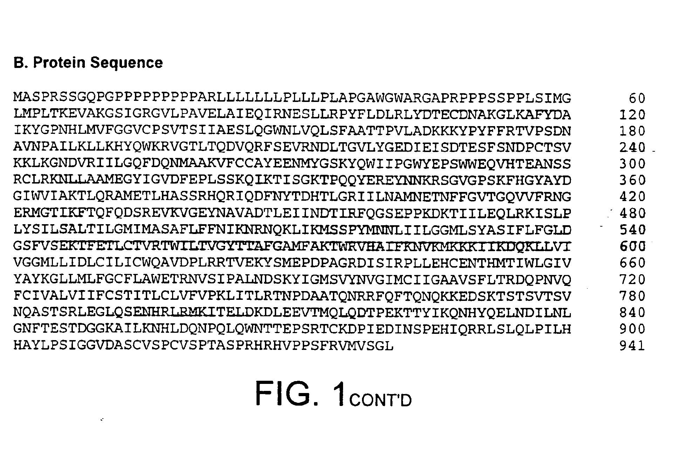

According to one embodiment of the invention there is provided a GABA_{B} receptor comprising a heterodimer between a GABA_{B}-R1 receptor protein and a GABA_{B} R2 receptor protein, wherein the GABA_{B}-R2 receptor protein has the amino acid sequence provided in Fig. 1B or an amino acid sequence that has at least 90 % homolgy to the amino acid sequence provided in Fig. 1B.

According to a further embodiment of the invention there is provided an expression vector comprising a nucleotide sequence encoding for a GABA_{B}-R1 receptor protein and a nucleotide sequence encoding for a GABA_{B}-R2 receptor protein, said vector being capable of expressing both GABA_{B}-R1 and GABA_{B}-R2 receptor proteins, wherein the GABA_{B}-R2 receptor protein has the amino acid sequence provided in Fig. 1B or an amino acid sequence having at least 90 % homology to the amino acid sequence provided in Fig. 1B.

According to another embodiment of the invention there is provided an isolated GABA_{B}-R2 receptor protein having the amino acid sequence provided in Fig. 1B.

According to a further embodiment of the invention, there is provided a nucleotide sequence encoding a GABA_{B}-R2 receptor protein of the invention, or a nucleotide sequence which is complementary thereto.

According to a further embodiment of the invention there is provided a nucleotide sequence encoding a GABA_{B}-R2 receptor as shown in Fig. 1A, or a nucleotide sequence which is complementary thereto.

According to a further embodiment of the invention there is provided an expression vector comprising a nucleotide sequence of the invention which is capable of expressing a GABA_{B}-R2 receptor protein.

According to a further embodiment of the invention there is provided a stable cell line comprising a vector of the invention.

According to a further embodiment of the invention there is provided a stable cell line modified to express both GABA_{B}-R1 and GABA_{B}-R2 receptor proteins, wherein the GABA_{B}-R2 receptor protein has the amino acid sequence provided in Fig. 1B or an amino acid sequence having at least 90 % homology to the amino acid sequence provided in Fig. 1B.

According to a further embodiment of the invention there is provided a GABA_{B} receptor comprising a heterodimer between a GABA_{B}-R1 receptor protein and a GABA_{B}-R2 receptor protein produced by a stable cell line of the invention.

According to a further embodiment of the invention, there is provided a GABA_{B}-R2 receptor protein produced by a stable cell line of the invention.

According to a further embodiment of the invention there is provided an antibody specific for a GABA_{B} receptor or receptor protein of the invention.

According to a further embodiment of the invention there is provided a method for identification of a compound which exhibits GABA_{B} receptor modulating activity, comprising contacting a GABA_{B} receptor of the invention with a test compound and detecting modulating activity or inactivity.

### Brief Description of the Figures

**Figure 1. Nucleotide and protein sequences of Human GABA**_{**B**}**-R2**
   Nucleotide sequence (a) and the translated protein sequence (b) for Human GABA_{B}-R2 are shown.
**Figure 2. Protein alignments between GABA**_{**B**}**-R1a, GABA**_{**B**}**-R1b, GABA**_{**B**}**-R1c splice variants and GABA**_{**B**}**-R2.**
   Amino-acid sequences of the human GABA_{B}-R1a, GABA_{B}-R1b and GABA_{B}-R2 receptors aligned for comparison. Signal sequences and predicted cleavage point ·( 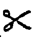 ), together with the N-terminal splice points for GABA_{B}-R1a and GABA_{B}-R1b are shown. GABA_{B}-R1c sequence is exactly that of GABA_{B}-R1a, except for the deletion of 63 amino acids (open box). Amino acids conserved between GABA_{B}-R1a and GABA_{B}-R1b are in bold type and potential N-glycosylation sites (*) are shown. Lines beneath the text show positions of the seven predicted TM domains and regions encoding coiled coil structure are indicated by shading. The C-terminal region of GABA_{B}-R1 used as the bait in the yeast two hybrid analysis is marked as 'BAIT→', and GABA_{B}-R2 C-terminal domains recovered from the library screen against GABA_{B}-R1 C-terminus are shown as 'YTH HITS→'.

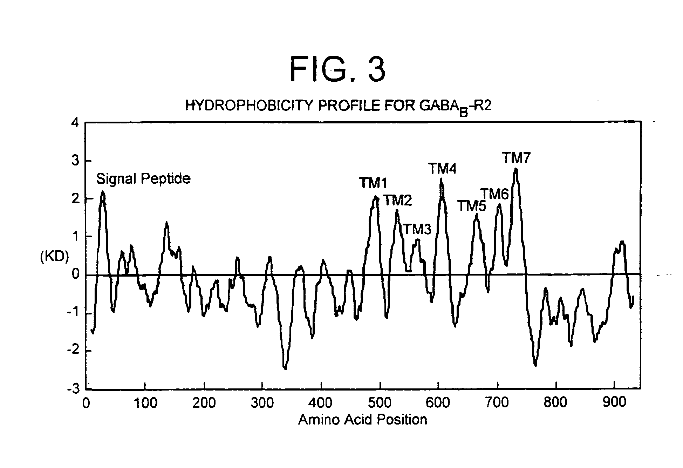

**Figure 3. Hydrophobicity profile of GABA**_{**B**}**-R2.**
   Hydrophobicity profiles of GABA_{B}-R2 sequence were determined using the Kyte-Doolittle algorithm, whereby positive values indicate hydrophobic regions. The predicted signal sequence and seven trans-membrane domains are shown.

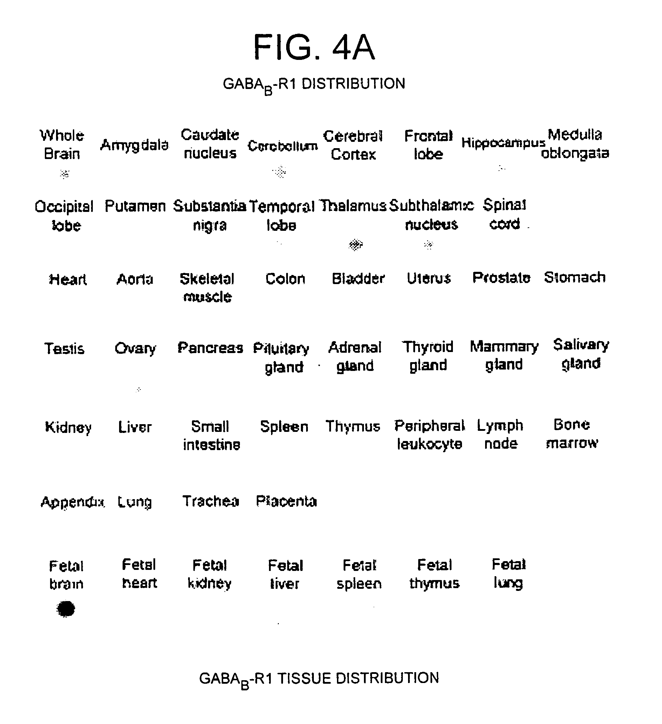

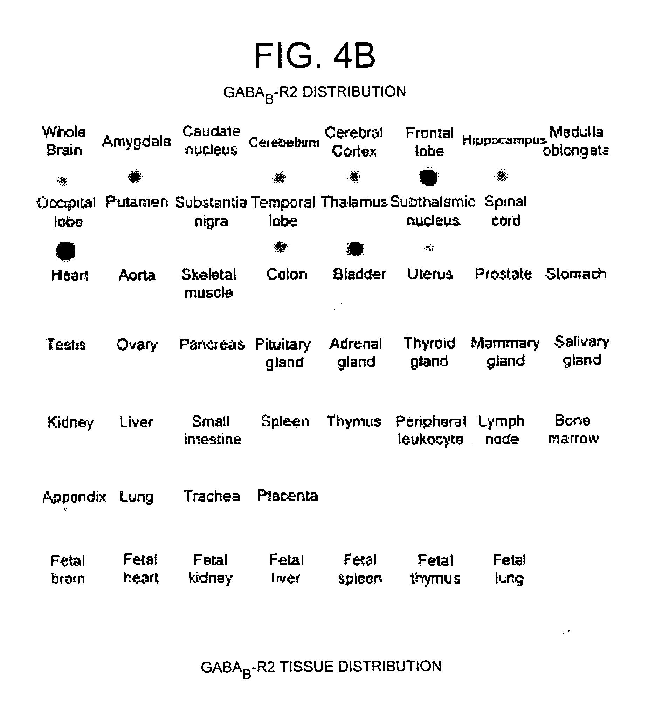

**Figure 4. Tissue Distribution Studies for Human GABA**_{**B**}**-R1 and GABA**_{**B**}**-R2.**
   A Human RNA Master Blot (Clontech), containing normalised polyA' mRNA from multiple tissues of adult and fetal origin, were probed sequentially with a pan specific probe for GABA_{B}-R1 (all splice variants) followed by a GABA_{B}-R2 specific probe. Resulting autoradiographic analysis of the blots are shown, together with a grid identifying tissue type. Specificity controls include yeast RNA and *E*. *coli* DNA.

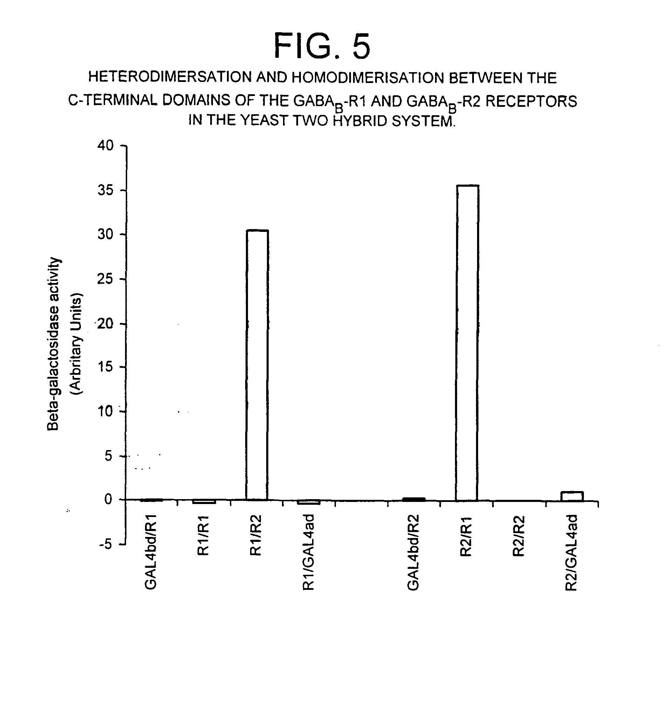

**Figure 5. Heterodimerisation and homodimerisation between the C-terminal domains of the GABA**_{**B**}**-R1 and GABA**_{**B**}**-R2 receptors in the yeast two hybrid system.**
   β-galactosidase activity was measured in yeast Y190 cells expressing the GABA_{B}-R1 or the GABA_{B}-R2 C-termini, either against empty vector or against each other in all combinations, using ONPG. Of each pair of proteins expressed in the two hybrid system, the first always refers to the GAL4_{BD} fusion construct whilst the second refers to the GAL4_{AD} fusion construct. β-galactosidase activity is determined relative to cell numbers and is in arbitary units.

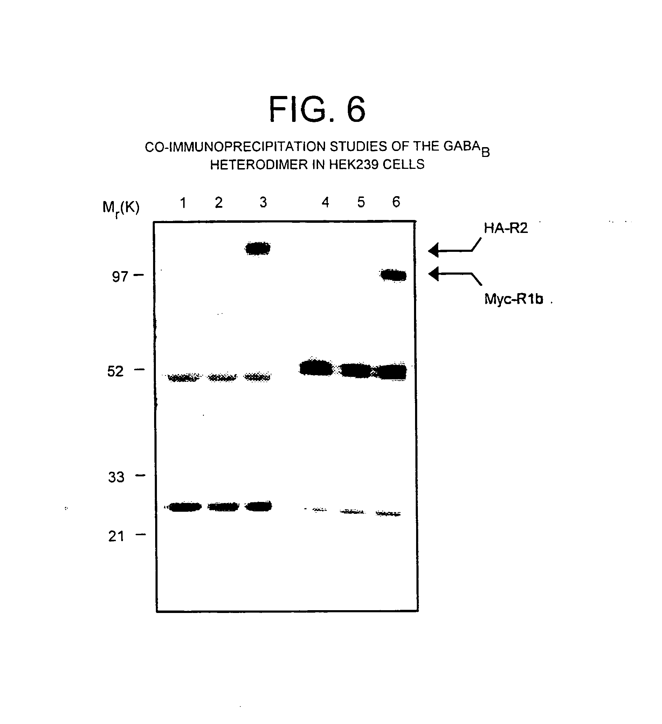

**Figure 6. Co-immunoprecipitation studies of the GABA**_{**B**} **heterodimer in HEK239 cells.**
   HEK293T cells were transfected with 1 µg each of either Myc-GABA_{B}-R1b or HA-GABA_{B}-R2 alone or in combination. Cells were harvested 48 h after transfection, lysed and epitope tagged receptors immunoprecipitated using 12CA5 (HA) or 9E10 (Myc) antisera as described in Methods. Immune complexes were then subjected to SDS-PAGE, transferred to nitrocellulose, and captured Myc-GABA_{B}-R1b and HA-GABA_{B}-R2 identified by immunoblotting with Myc and HA, respectively. Lanes 1 and 4, immunoprecipitates of cells transfected with Myc-GABA_{B}-R1b only; lanes 2 and 5, HA-GABA_{B}-R2 only; lanes 3 and 6, immunoprecipitates of cells transfected with Myc-GABA_{B}-R1b together with HA-GABA_{B}-R2. Lanes 1-3, lysates immunoprecipitated with 9E10 (Myc) and blotted to 12CA5(HA); lanes 4-6, lysates immunoprecipitated with 12CA5(HA) and blotted with 9E10 (Myc)

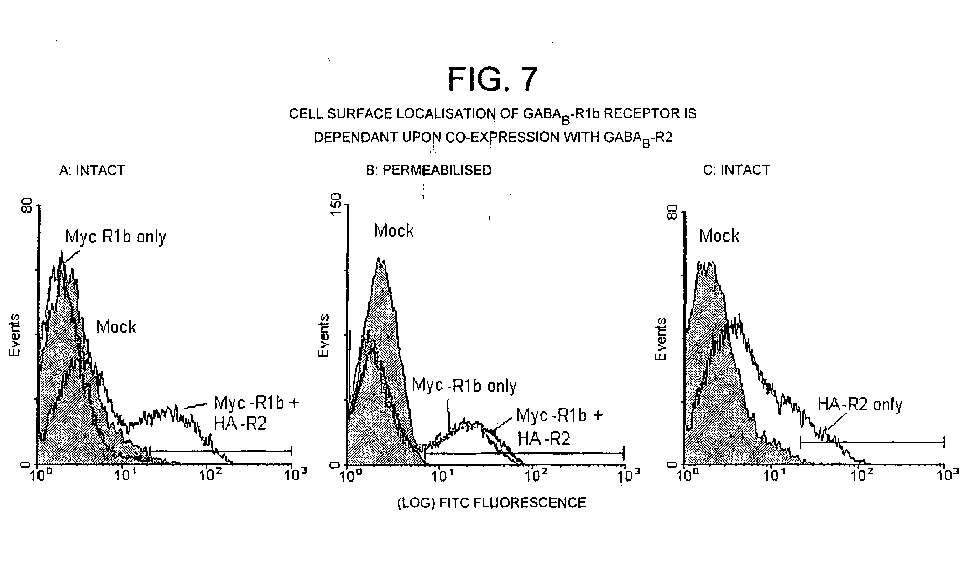

**Figure 7. Cell surface localisation of GABA**_{**B**}**-R1 receptor is dependent upon coexpression with GABA**_{**B**}**-R2.**
   Flow cytometry was performed on HEK293T cells transfected with 1µg of either Myc-GABA_{B}-R1b or HA-GABA_{B}-R2 or both receptors in combination. (**A**) Analysis using 9E10 (c-Myc) as primary antibody to detect Myc-GABA_{B}-R1b; intact cells. (**B**) Analysis using 9E10 (c-Myc) as primary antibody to detect Myc-GABA_{B}-R1b; permeabilised cells. (**C**) Analysis using 12CA5 (HA) as primary antibody to detect HA-GABA_{B}-R2; intact cells. Mock transfected cells, reflecting background fluorescence, are shaded and the marker indicates fluorescence measured over background levels. Myc-GABA_{B}-R1b data is shown as a grey line whereas co-expression of Myc-GABA_{B}-R1b with HA-GABA_{B}-R2 is shown in black. 30,000 cells were analysed in each sample. Histograms shown are from a single experiment. Quoted statistics are from mean of three separate transfections and analysis.

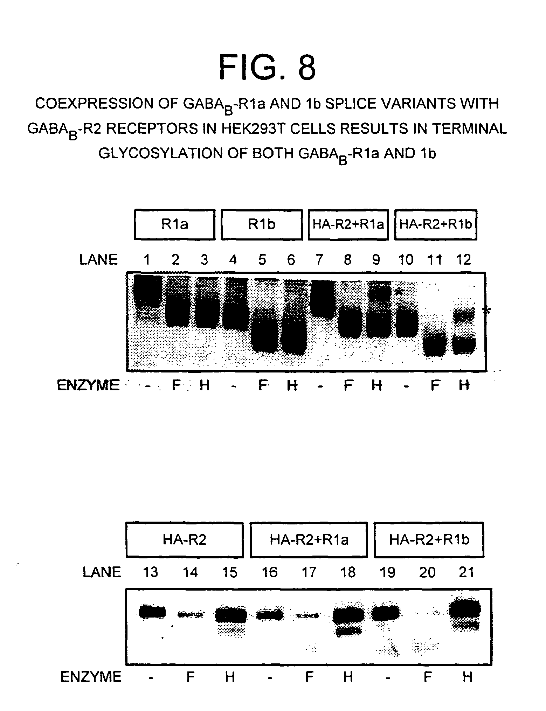

**Figure 8. Coexpression of GABA**_{**B**}**-R1a and 1b splice variants with GABA**_{**B-**}**R2 receptors in HEK293T cells results in terminal glycosylation of both GABA**_{**B**}**-R1 a and GABA**_{**B**}**-R1b.**
   P2 membrane fractions were derived from HEK293T cells that were transfected with 1 µg of either GABA_{B}-R1a (lanes 1-3), GABA_{B}-R1b (lanes 4-6) or HA-GABA_{B}-R2 (lanes 13-15), or with 1 µg each of HA-GABA_{B}-R2 in combination with 1 µg of either GABA_{B}-R1a (lanes 7-9, 16-18) or GRBA_{B}-R1b (lanes 10-12, 19-21). Glycosylation status of transfected receptors was assessed following treatment of P2 fractions (50 µg of membrane protein) with either vehicle (lanes 1, 4, 7, 10, 13, 16 and 19), endoglycosidase F (lanes 2, 5, 8, 11, 14, 17 and 20) or endoglycosidase H (lanes 3, 6, 9, 12, 15, 18 and 21). Samples were resolved by SDS-PAGE (10 % (w/v) acrylamide), transferred to nitrocellulose, and immunoblotted. Upper panel, antiserum 501 was used as primary reagent to allow identification of both GABA_{B}-R1a and 1b. Lower panel, 12CA5 anti-HA antiserum was employed to identify HA-GABA_{B}-R2. ^{*}, denotes terminally glycosylated forms of GABA_{B}-R1a and 1 b.

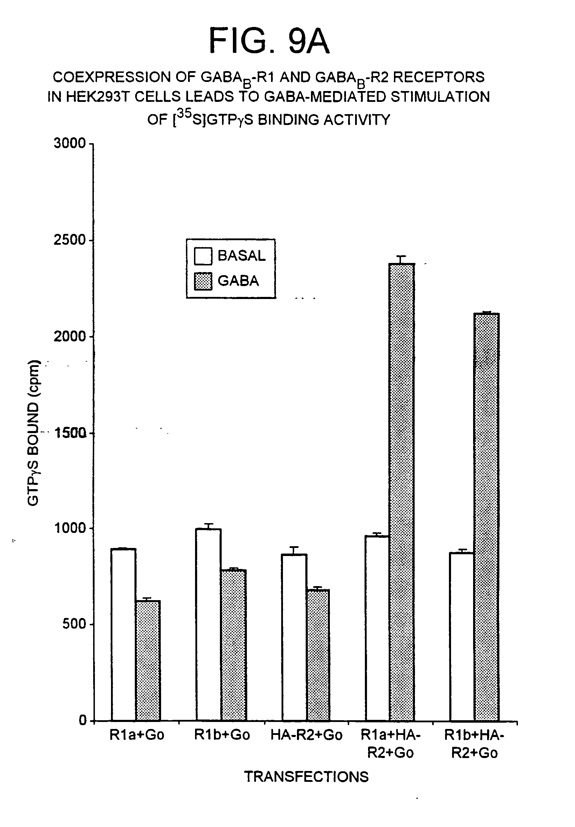

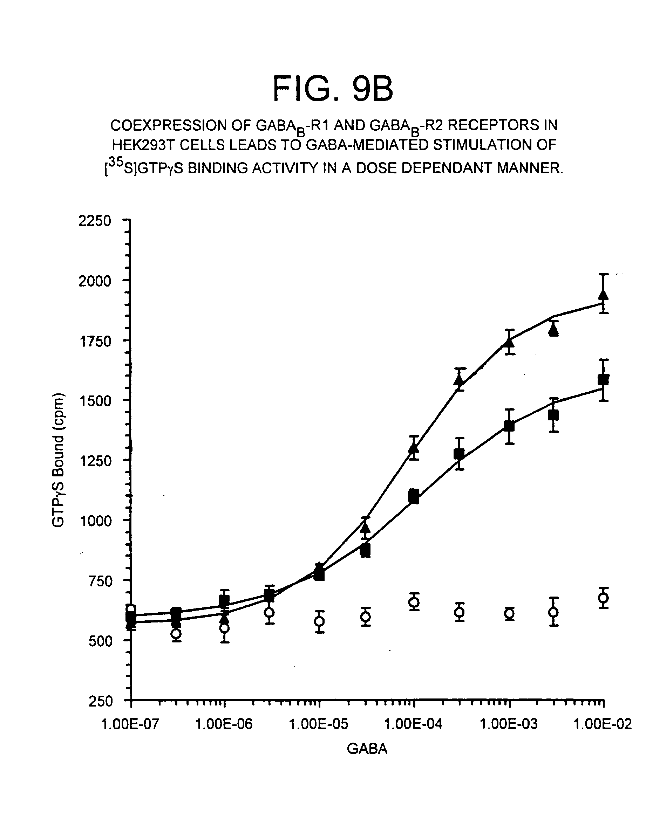

**Figure 9. Coexpression of GABA**_{**B**}**-R1 and GABA**_{**B**}**-R2 receptors in HEK293T cells leads to GABA-mediated stimulation of [**^{**35**}**S]GTPγS binding activity.**
   [³⁵S]GTPγS binding activity was measured on P2 particulate fractions derived from HEK293T cells transfected with 1 µg of Gₒ₁α together with 1 µg of either GABA_{B}-R1a, GABA_{B}-R1b or HA-GABA_{B}-R2; or with 1 µg each of Gₒ₁α and HA-GABA_{B}-R2 in combination with 1 µg of either GABA_{B}-R1a or GABA_{B}-R1b. **(A)** [³⁵S]GTPγS binding was measured in the absence (open bars) or presence (hatched bars) of GABA (10 mM) as described in Methods. (**B**) The ability of varying concentrations of GABA to stimulate the binding of [³⁵S]GTPγS was measured on P2 membrane fractions from HEK293T cells expressing either Gₒ₁α and HA-GABA_{B}-R2 alone (open circles) or in combination with either GABA_{B}-R1a (closed squares) or GABA_{B}-R1b (closed triangles). The data shown are the means ± S.D. of triplicate measurements and are representative of three independent experiments.

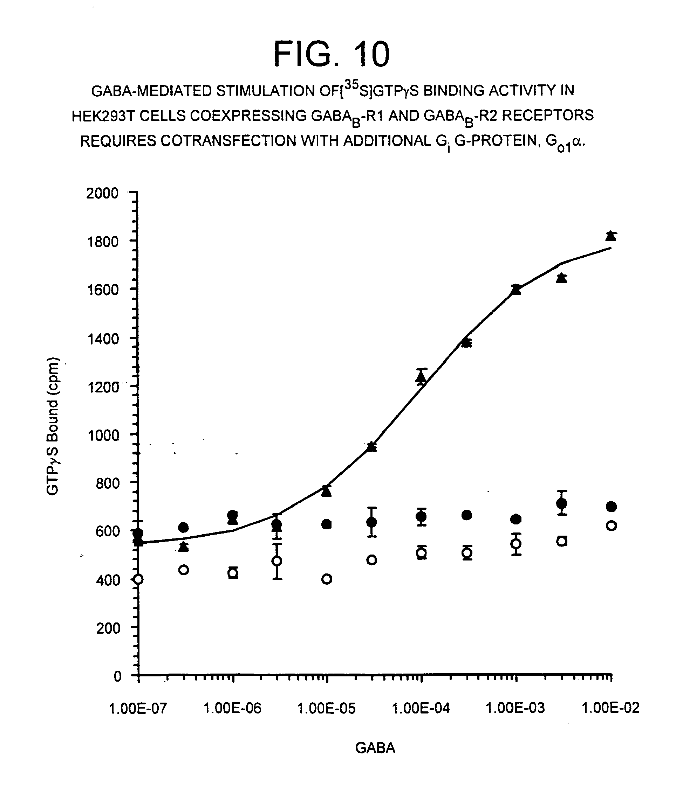

**Figure 10. GABA-mediated stimulation of [**^{**35**}**S]GTPγS binding activity in HEK293T cells coexpressing GABA**_{**B**}**-R1 and GABA**_{**B**}**-R2 receptors requires cotransfection with additional G**_{**i**} **G protein, G**_{**o1**}**α.**
   [³⁵S]GTPγS binding activity was measured on P2 particulate fractions derived from HEK293T cells transfected with HA-GABA_{B}-R1b (1 µg) together with HA-GABA_{B}-R2 (1µg) and Gₒ₁α (1µg) (closed triangles), or in combination with either HA-GABA_{B}-R2 (1µg) (open circles) or Gₒ₁α (1 µg) (closed circles). The ability of varying concentrations of GABA to stimulate the binding of [³⁵S]GTPγS was determined. Data shown are the mean ± S.D. of triplicate measurements.

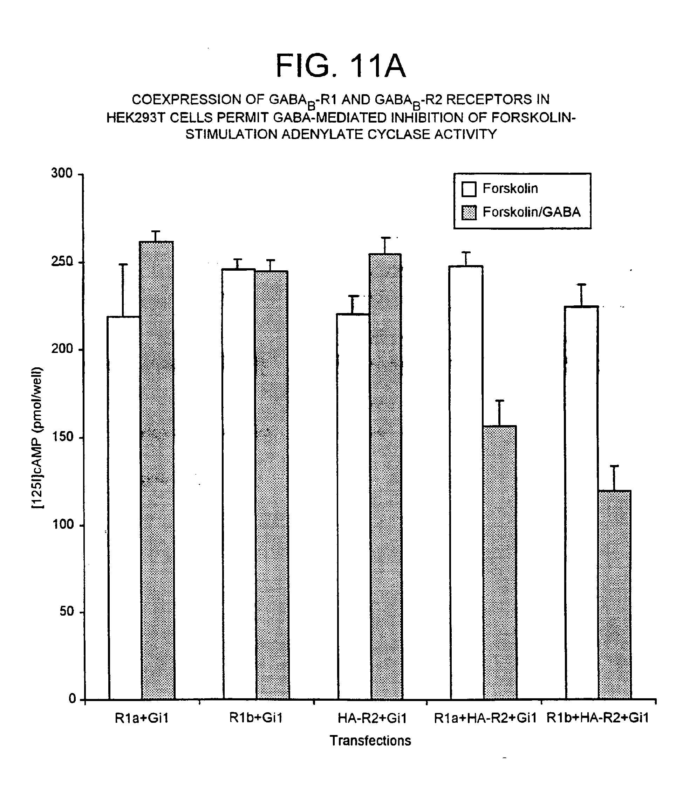

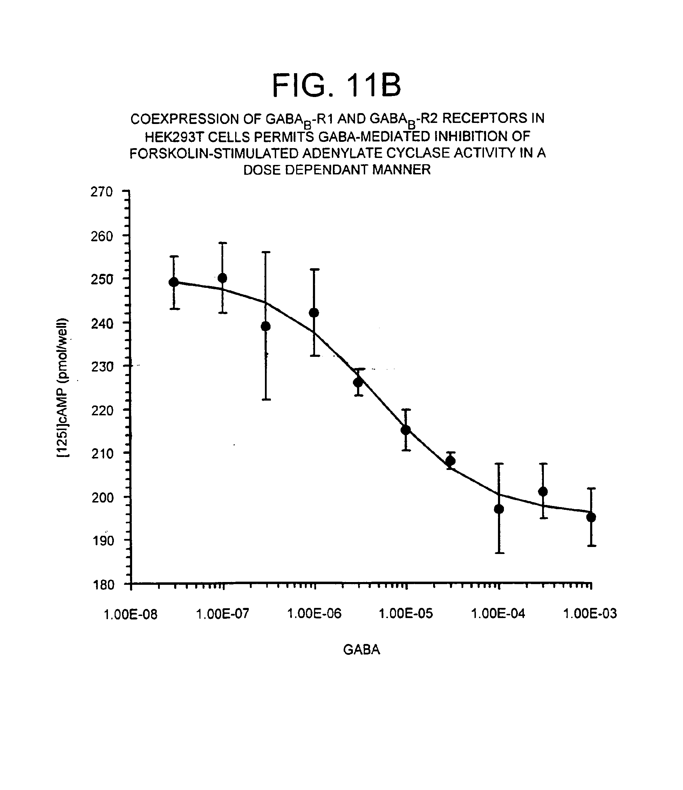

**Figure 11. Coexpression of GABA**_{**B**}**-R1 and GABA**_{**B**}**-R2 receptors in HEK293T cells permits GABA-mediated inhibition of forskolin-stimulated adenylate cyclase activity**
   cAMP levels were measured in HEK293T cells transfected with 1 µg of Gᵢ₁α together with 1 µg of either GABA_{B}-R1a, GABA_{B}-R1b or HA-GABA_{B}-R2; or with 1 µg each of Gᵢ₁α and HA-GABA_{B}-R2 in combination with 1 µg of either GABA_{B}-R1a or GABA_{B}-R1b, as described in Methods. (**A**) cAMP levels were determined in cells treated with forskolin (50 µM) in the absence (open bars) or presence (hatched bars) of GABA (1 mM). (**B**) ability of varying concentrations of GABA to inhibit forskolin-elevated adenylate cyclase activity in HEK293T cells expressing Gᵢ₁α and HA-GABA_{B}-R2 in combination with GABA_{B} R1b. The data shown are the means ± S.D. of triplicate measurements.

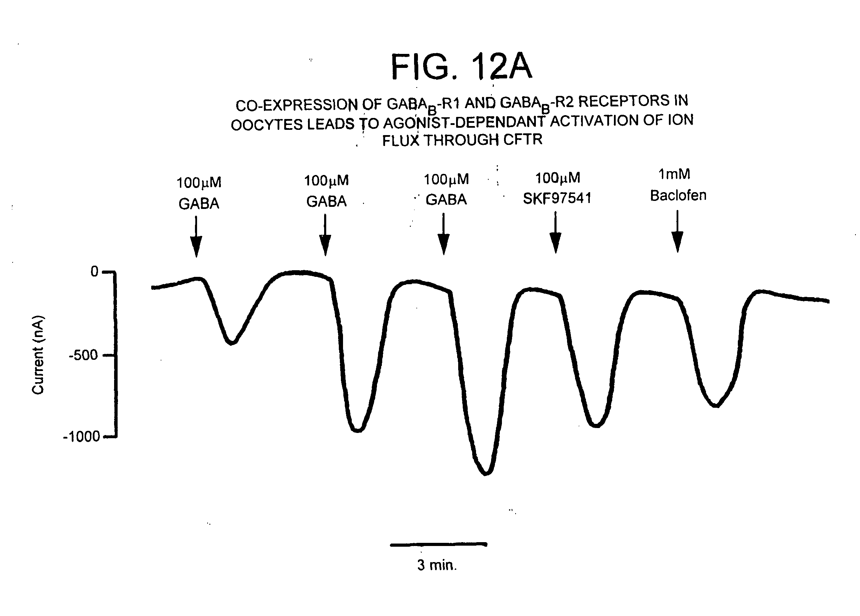

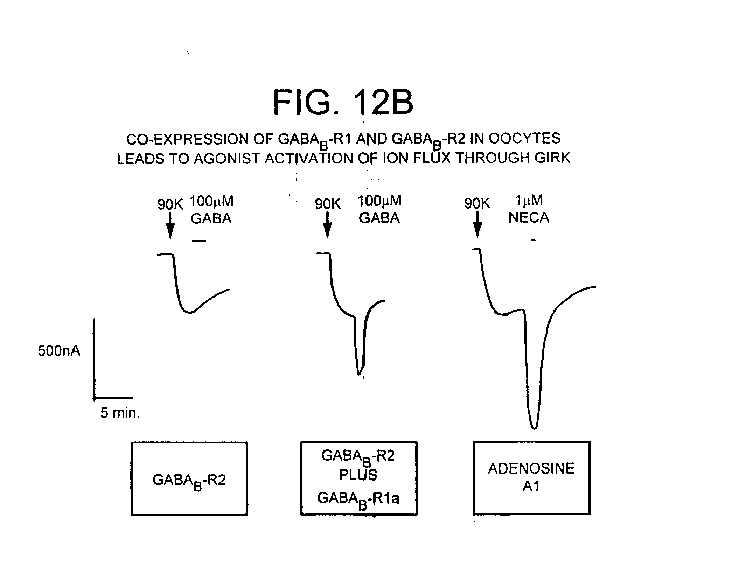

**Figure 12. Co-expression of GABA**_{**B**}**-R1 and GABA**_{**B**}**-R2 receptors in *Xenopus* oocytes permits agonist-dependant activation of ion flux through CFTR and GIRK1/4.**
   *Xenopus* oocytes were injected with cRNA encoding GABA_{B}-R1 and GABA_{B}-R2 receptors (in equal amounts for CFTR, 1:2 ratio for GIRK) plus either CFTR (A) or the GIRK1/GIRK4 heteromer (B). A, Time course plot for an oocyte expressing GABA_{B}-R1, GABA_{B}-R2 and CFTR. Application of 100mM GABA, 100mM SKF97541 or 1mM Baclofen (arrows) activated a large inward CFTR current. Note the increase in CFTR response seen with repeated GABA application. B, Time course plot for an oocyte expressing GABA_{B}-R1, GABA_{B-}R2, GIRK1 and GIRK4. Switching from ND96 (low potassium) to 90K (high potassium) solution led to an inward shift in holding current, showing that the GIRK1/GIRK4 channel is expressed in this oocyte. Subsequent application of 100mM GABA activated a large inward current (middle panel). Negative and positive control experiments are shown from oocytes expressing the GABA_{B}-R2 receptor alone (left panel) and those expressing the adenosine A1 receptor (right panel).

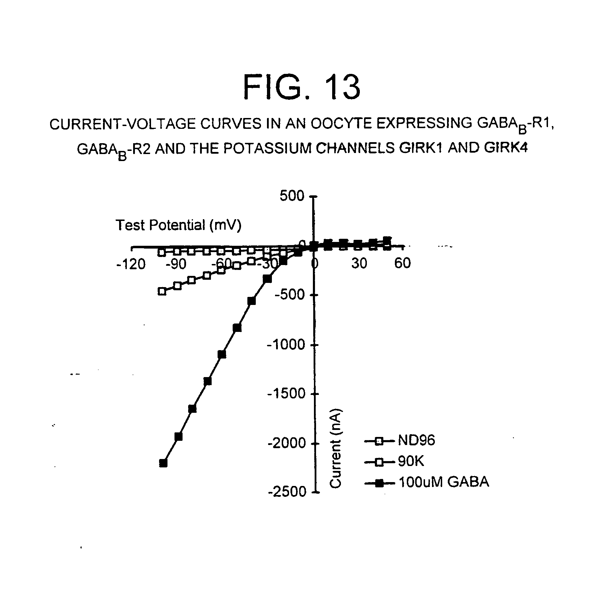

**Figure 13. Current-voltage curves in an oocyte expressing GABA**_{**B**}**-R1, GABA**_{**B**}**-R2 and the potassium channels GIRK1 and GIRK4.**
   Current-voltage curves are shown for a single oocyte following application of 200ms voltage-clamp pulses from a holding potential of -60mV to test potentials between -100mV and +50mV. Steady-state current is plotted against test potential in ND96 solution (low potassium), 90K solution (90mM potassium) and 90K plus 100mM GABA. Note the basal GIRK1/4 current recorded in 90K solution and the large agonist-evoked activation of the GIRK potassium channel.

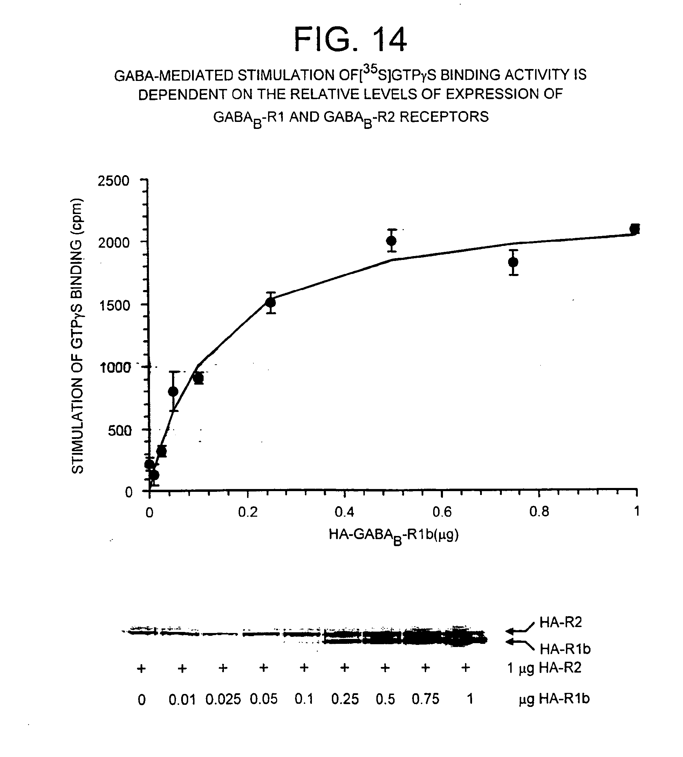

**Figure 14. GABA-mediated stimulation of [**^{**35**}**S]GTPγS binding activity is dependent on the relative levels of expression of GABA**_{**B**}**-R1 and GABA**_{**B**}**-R2 receptors**
   HEK293T cells were transfected with HA-GABA_{B}-R2 (1µg) and Gₒ₁α (1µg) together with various amounts (0-1 µg) of HA-GABA_{B}-R1 b. Cells were harvested 48 h after transfection and P2 membrane fractions were prepared. (**A**) Agonist stimulation of [³⁵S]GTPγS binding activity measured in transfected cell membranes in the presence of GABA (10 mM). Data are shown as stimulation above basal (cpm) and are the mean ± S.D. of triplicate measurements. (**B**) Cell membranes were immunoblotted with anti-HA antiserum to allow the relative levels of HA-GABA_{B}-R2 and HA-GABA_{B}-R1b receptors to be evaluated.

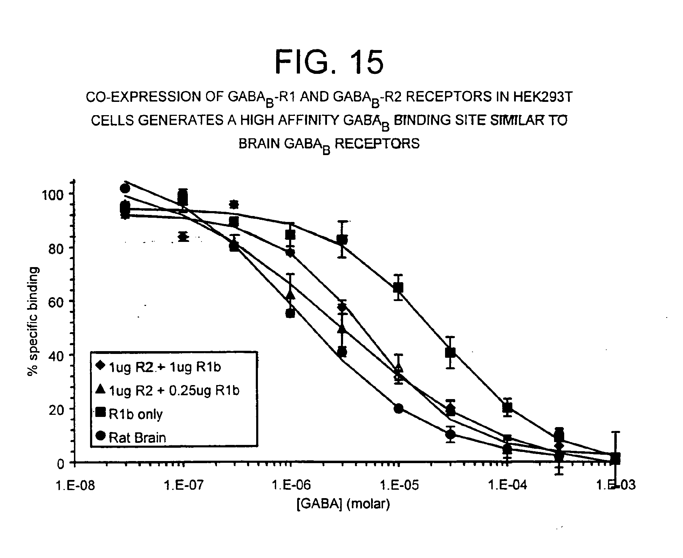

**Figure 15. Co-expression , of GABA**_{**B**}**-R1 and GABA**_{**B**}**-R2 receptors in HEK293T cells generates a high affinity GABA**_{**B**} **binding site similar to brain GABA**_{**B**} **receptors.**
   P2 membrane fractions were prepared from HEK 293T cells transfected using the same conditions described for GTPγS binding studies. % specific binding was determined for the displacement of [3H]-CGP54626 by GABA. Data shown are the mean of minimum of triplicate studies ± sem.

### Detailed Description of the Invention

Throughout the present specification and the accompanying claims the words "comprise" and "include" and variations such as "comprises", "comprising", "includes" and "including" are to be interpreted inclusively. That is, these words are intended to convey the possible inclusion of other elements or integers not specifically recited, where the context allows.

As previously explained, the present invention includes a number of important aspects. In particular the present invention relates to isolated GABA_{B}-R2 receptor proteins and GABA_{B} receptors comprising an heterodimer between a GABA_{B}-R1 receptor protein and a GABA_{B}-R2 receptor protein as well as other related aspects. In the context of the present invention the wording "isolated" is intended to convey that the receptor protein is not in its native state, insofar as it has been purified at least to some extent or has been synthetically produced, for example by recombinant methods. The term "isolated" therefore includes the possibility of the receptor protein being in combination with other biological or non-biological material, such as cells, suspensions of cells or cell fragments, proteins, peptides, organic or inorganic solvents, or other materials where appropriate, but excludes the situation where the receptor protein is in a state as found in nature.

Routine methods, as further explained in the subsequent experimental section, can be employed to purify and/or synthesise the receptor proteins according to the invention. Such methods are well understood by persons skilled in the art, and include techniques such as those disclosed in Sambrook, J. et al, 1989, The present invention not only includes the GABA_{B}-R2 receptor protein having the amino acid sequence shown in Fig. 1B, but also GABA_{B}-R2 receptor proteins having an amino acid sequence that has at least 90% homology to the amino acid sequence shown in Fig. 1B. Such proteins retain the same essential character of the GABA_{B-}R2 receptor protein for which sequence information is provided. For example, proteins with at least 95% homology with the amino acid sequences provided in Fig. 1B are considered as GABA_{B}-R2 receptor proteins. Such proteins may include the deletion, modification or addition of single amino acids or groups of amino acids within the amino acid sequence, as long as the biological functionality of the protein is not adversely affected.

The invention also includes nucleotide sequences which encode for GABA_{B}-R2 receptor proteins as well as nucleotide sequences which are complementary thereto. Preferably the nucleotide sequence is a DNA sequence and most preferably, a cDNA sequence.

The present invention also includes expression vectors which comprise nucleotide sequences encoding for the GABA_{B}-R2 receptor proteins. A further aspect of the invention relates to an expression vector comprising nucleotide sequences encoding for a GABA_{B}-R1 receptor protein and a GABA_{B}-R2 receptor protein. Such expression vectors are routinely constructed in the art of molecular biology and may involve the use of plasmid DNA and appropriate initiators, promoters, enhancers and other elements, which may be necessary, and which are positioned in the correct orientation, in order to allow for protein expression.

The invention also includes cell lines which have been modified to express the novel receptor. Such cell lines include transient, or preferably stable higher eukaryotic cell lines, such as mammalian cells or insect cells, lower eukaryotic cells, such as yeast or prokaryotic cells such as bacterial cells. Particular examples of cells which have been modified by insertion of vectors encoding for the receptor proteins according to the invention include HEK293T cells and oocytes. Preferably the cell line selected will be one which is not only stable, but also allows for mature glycosylation and cell surface expression of the inventive receptors. In the case of the functional GABA_{B} receptor which comprises a heterodimer of GABA_{B}-R1 and GABA_{-B}-R2 subunits, the cell line may include a single vector which allows for expression of both of the receptor subtypes, or alternatively separate vectors for each subunit. It is preferred however, that the receptor subtypes should be co-expressed in order to optimise the dimerisation process, which will result in full glycosylation and transport of the glycosylated dimer to the cell surface.

It is also possible for the receptors of the invention to be transiently expressed in a cell line or on a membrane, such as for example in a baculovirus expression system. Such systems, which are adapted to express the receptors according to the invention, are also included within the scope of the present invention.

A particularly preferred aspect of the invention is the heterodimer formed between the GABA_{B}-R1 and GABA_{B}-R2 receptor proteins which results in the formation of a functional GABA_{B} receptor. Without wishing to be bound by theory, it appears that the formation of the heterodimer takes place via the coiled-coil domains within the receptor C-terminal tails, and that this in turn is a pre-requisite for transport and full glycosylation of a GABA_{B}-R1, and also for generation of an high affinity GABA_{B} receptor at the cell surface.

The heterodimer which forms a functional GABA_{B} receptor can comprise any GABA_{B}-R1 receptor subtype or splice variant. Although we are presently only aware of only one GABA_{B}-R2 subtype, it is envisaged that the heterodimers according to the present invention can include other GABA_{B}-R2 subtypes or splice variants such as GABA_{B}-R2 receptor proteins having at least 90% homonology to the GABA_{B}-R2 receptor protein shown in Figure 1B.

In particular, the functional GABA_{B} receptor may include GABA_{B}-R1 receptor proteins selected from GABA_{B}-R1a, GABA_{B}-R1b, GABA_{B}-R1c splice variants, variants thereof or even other GABA_{B}-R1 receptor subtypes or splice variants which have not yet been identified.

According to another aspect, the present invention also relates to antibodies which have been raised by standard techniques and are specific for the receptor proteins according to the invention. Such antibodies could for example, be useful in purification, isolation or screening involving immuno precipitation techniques and may be used as tools to further ellucidate GABA_{B} receptor function, or indeed as therapeutic agents in their own right. Antibodies may also be raised against specific epitopes of the receptors according to the invention, as opposed to the monomer subunits.

An important aspect of the present invention is the use of receptor proteins according to the invention, particularly the heterodimer GABA_{B} receptor, in screening methods designed to identify compounds which act as receptor ligands and which may be useful to modulate receptor activity. In general terms, such screening methods will involve contacting the receptor protein concerned, preferably the heterodimeric GABA_{B} receptor, with a test compound and then detecting modulation in the receptor activity, or indeed detecting receptor inactivity, which results. The present invention also includes within its scope those compounds which are identified as possessing useful GABA_{B} receptor modulation activity, by the screening methods referred to above. The screening methods comprehended by the invention are generally well known to persons skilled in the art, and are further discussed in the experimental section which follows.

Another aspect of the present invention is the use of compounds which have been identified by screening techniques referred to above in the treatment or prophylaxis of disorders which are responsive to modulation of a GABA_{B} receptor activity, in a mammal. By the term "modulation" what is meant is that there will be either agonism or antagonism at the receptor site which results from ligand binding of the compound at the receptor. GABA_{B} receptors have been implicated in disorders of the central nervous system (CNS), gastrointestinal (Gl) tract, lungs and bladder and therefore modulation of GABA_{B} receptor activity in these tissues will result in a positive therapeutic outcome in relation to such disorders. In particular, the compounds which will be identified using the screening techniques according to the invention will have utility for treatment and/or prophylaxis of disorders such as spasticity, epilepsy, Alzheimer's disease, pain as well as affective disorders and feeding disorders. It is to be understood however, that the mention of such disorders is by way of example only, and is not intended to be limiting on the scope of the invention.

The compounds which are identified according to the screening methods outlined above may be formulated with standard pharmaceutically acceptable carriers and/or excipients as is routine in the pharmaceutical art, and as fully described in Remmington's Pharmaceutical Sciences, Mack Publishing Company, Eastern Pennsylvania, 17th Ed, 1985, the disclosure of which is included herein in its entirety by way of reference.

The compounds may be administered via enteral or parenteral routes such as via oral, buccal, anal, pulmonary, intravenous, intraarterial, intramuscular, intraperitoneal, topical or other appropriate administration routes.

Other aspects of the present invention will be further explained, by way of example, in the appended experimental section.

### Experimental

### Results

### 1. Cloning of Human GABA_{B}-R1 and a novel Receptor subtype, GABA_{B}-R2

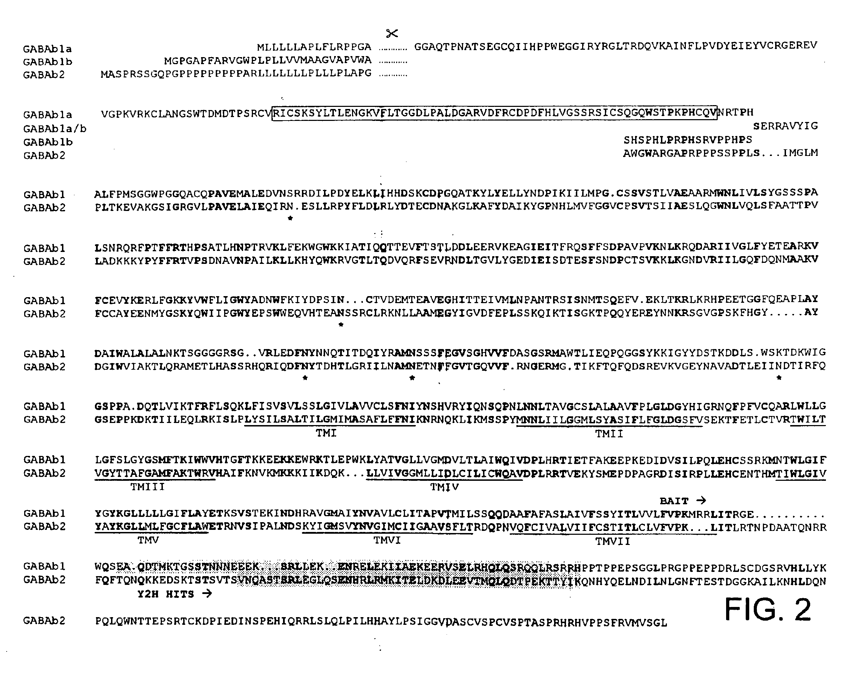

Human homologues to the rat GABA_{B}-R1a and 1 b splice variants were identified from ESTs and subcloned from Human cerebellum cDNA, using a combination of PCR and Rapid amplification of cDNA ends (RACE) PCR. Human GABA_{B-}R1 a and 1b sequences reveal over 99% identity to the rat GABA_{B}-R1a and GABA_{B}-R1b (data not shown). These receptors, like their rat counterparts, both have signal sequences, followed by extended N-termini, a typical seven-transmembrane topology and short intracellular C-terminal tail. The N-terminus encodes the GABA binding domain, which is predicted by limited homology to bacterial periplasmic proteins to exist as two globular domains that capture GABA (Bettler *et al.,* 1998), as well as three potential *N*-glycosylation sites. Interestingly the GABA_{B}-R1a splice variant N-terminus encodes 129 amino acids over that of GABA_{B}-R1b, which encode two tandem copies of the 'short consensus repeat' or sushi domain. Sushi domains are approximately 60 amino acids in length and exist in a wide range of proteins involved in complement and cell-cell adhesion (Chou and Heinrikson, 1997). Therefore the sushi domains within GABA_{B}-R1a may direct protein-protein interactions, possibly through cell-cell contact and may reflect a further role for GABA_{B}-R1a, over and above that of GABA_{B}-R1 b. Interestingly during the isolation of these clones, a novel N-terminal splice variant, GABA_{B}-R1c was identified. GABA_{B}-R1c differs from GABA_{B}-R1a by a 185bp deletion from bases 290 to 475 (see Figure 2). This region encodes one of the two Sushi domains unique to GABA_{B}-R1 a and therefore the GABA_{B-}R1a and GABA_{B}-R1c splice variants, together with their cellular localisation, may be significant in the biology of GABA_{B} receptors. Indeed, *in situ* hybridisations suggest that GABA_{B}-R1a and GABA_{B}-R1b have different sub-cellular localisations, with GABA_{B}-R1a expressed at pre-synaptic rather than at post-synaptic sites (Bettler *et al.,* 1998).

Database searches also identified a number of ESTs showing weaker homology to GABA_{B}-R1, suggesting the existence of a novel GABA_{B} receptor subtype. Using PCR on Human Brain cerebellum cDNA, we confirmed the existence of such a novel GABA_{B} receptor which we cloned and sequenced (Figure 1). This novel receptor, which we have called GABA_{B}-R2, shows an overall 54% similarity and 35% identity to GABA_{B}-R1 over the full length of the protein (Figure 2). As expected, hydrophobicity profiles for GABA_{B}-R2 (Figure 3) suggested that the protein has a 42 amino acid signal peptide followed by an extracellular N-terminal domain comparable in size to that of GABA_{B}-R1b and seven membrane spanning regions. In total five N-glycosylation sites were predicted over the N-terminal domain, three of which are conserved within GABA_{B}-R1. Finally, the receptor encodes an intracellular C-terminal domain, which is considerably larger than that of GABA_{B}-R1. No sushi domains were identified within GABA_{B}-R2 sequence and we have no evidence for any splice variants to date.

### 2. Tissue Distribution

Expression levels of both GABA_{B}-R1 and GABA_{B}-R2 were determined and compared in different tissues and developmental stages by probing Human RNA Master Blots (Clontech). These blots contain polyA⁺ RNA samples from 50 human tissues that have been normalized to the mRNA expression levels of eight different "housekeeping" genes. GABA_{B}-R1 levels were examined using a pan-specific probe covering all splice variants (Figure 4a) and the blots indicate that in accordance with the observations of Kaupmann *et al.,* (1997), GABA_{B}-R1 is highly expressed in the CNS, in all areas of the brain and spinal cord. However, in contrast to Kaupmann *et al.,* (1997), we find that GABA_{B}-R1 is also expressed at comparable levels in peripheral tissues, with particularly high levels of expression in the pituitary, lung, ovary, kidney, small intestine, and spleen. In marked contrast, GABA_{B}-R2 is specifically expressed at high levels only in the CNS, with the possible exception of spinal cord where expression appears somewhat lower. No signal is seen for peripheral tissues, in either adult or fetal tissues (Figure 4b). This markedly different distribution of mRNA levels between GABA_{B}-R1 and GABA_{B}-R2 suggests that the two subtypes may have distinct roles in the CNS and periphery.

### 3. Initial Expression studies.

We reasoned that GABA_{B}-R2 could be a high affinity GABA_{B} receptor and therefore, expressed the receptor in both *Xenopus* oocytes and HEK293T cells and looked for functional responses. However, despite repeated attempts, we were unable to detect any functional activation of GABA_{B}-R2 or indeed, GABA_{B-}R1a, GABA_{B}-R1b or GABA_{B}-R1c receptors by either GABA itself or GABA_{B} selective agonists (See Figures 9, 11 and 12). Several lines of evidence clearly indicated that GABA_{B}-R1 was not expressed as predicted *in vivo.* Firstly, flow cytometry of HEK293T cells, expressing GABA_{B}-R1b, revealed that receptors were retained on internal membranes rather than expressed at the cell surface (Figure 7). Secondly, GABA_{B}-R1a and GABA_{B}-R1b were expressed as immature glycoproteins, by virtue of their sensitivity to endoglycosidases F and H (Figure 8, lanes 1-6) and finally, GABA_{B}-R1 co-expression in oocytes with either GIRK or CFTR, gave no indication of a functional response (data not shown). We concluded that some additional co-factor must be required to promote a functional response.

### 4. Yeast Two Hybrid library screening

The calcitonin-receptor like receptor is retained as an immature glycoprotein within the endoplasmic reticulum and requires an accessory protein from the recently identified RAMP protein family to transport the receptor to the surface to generate a functional CGRP (Calcitonin gene-related peptide) or adrenomedullin receptor (McLatchie *et al.,* 1998). We anticipated that GABA_{B}-R1 receptors should require an analogous trafficking factor or some other protein co-factor for its transport to the cell surface to generate a high affinity receptor. To identify such potential interacting proteins, a yeast two hybrid library screen was run using the C-terminal 108 amino acids of GABA_{B}-R1 against a Human Brain cDNA library. Interestingly, motif searches revealed a strong coiled-coil domain within these 108 residues, a structure known to mediate protein-protein interactions (Lupas, 1996). From a total of 4.3 x 10⁶ cDNAs, 122 positives hits were recovered, 33 of which encoded the whole C-terminal domain of GABA_{B-}R2. This domain of the GABA_{B}-R2 is likewise predicted to contain a coiled-coil motif, which aligns exactly with that of GABA_{B}-R1 (see Figure 2). This observation strongly suggests that the two receptors interact via their C-termini to form a heterodimer. Significantly, the screen did not retrieve the C-terminal domain of the GABA_{B}-R1 itself, implying that GABA_{B}-R1 is unable to homodimerise. This interaction was tested directly in the yeast two hybrid system using the C-termini of the two receptors (Figure 5). GABA_{B}-R1 and GABA_{B}-R2 were able to strongly interact via their C-termini, whilst neither receptor was able to homodimerise. This observation suggested that GABA_{B}-R1 and GABA_{B}-R2 form heterodimers via their C-terminal coiled-coil domains and led to speculation that homodimerisation may bring about a functional binding site *in vivo.* Therefore, we next confirmed the interaction between the two receptor subtypes by immunoprecipitation studies upon whole epitope-tagged receptor in transfected HEK293T cells.

### 5. Co-immunoprecipitation Studies.

Epitope tagged receptors, Myc-GABA_{B}-R1 b and HA-GABA_{B}-R2 were transiently expressed in HEK293T cells either alone or in combination. Immunoprecipitation of Myc-GABA_{B}-R1b from detergent-solubilised cell fractions with Myc antisera led to immunodetection of HA-GABA_{B}-R2 within immune complexes using HA as the primary antibody, but only upon receptor co-expression (Figure 6, lanes 1-3). GABA_{B}-R1 and GABA_{B}-R2 association was confirmed by co-immunodetection of Myc-GABA_{B}-R1b from immune complexes captured using the anti-HA antibody. Once again, co-immunoprecipitation could only be seen when the two receptor forms were co-expressed (Figure 6, lanes 4-6). Hence in agreement with the yeast two hybrid observations, these data provide compelling evidence for heterodimerisation between full-length expressed GABA_{B}-R1 and GABA_{B}-R2 in mammalian cells. Therefore, we next examined GABA_{B} receptor responses following co-expression of both receptor subtypes in HEK293T cells or in *Xenopus* oocytes.

### 6. Surface Expression of the Heterodimer

HEK293T cells were transiently transfected with Myc-GABA_{B}-R1b alone or in combination with HA-GABA_{B}-R2 and transfectants analysed by flow cytometry (Figure 7). Myc-immunoreactivity could not be detected on the surface of cells transfected with Myc-GABA_{B}-R1b alone (Figure7a), although cell permeabilisation revealed immunoreactivity in 35% (n=3) of the cell population (Figure 7b). This latter observation indicated that cells were efficiently transfected and suggested that expressed Myc-GABA_{B}-R1 receptors were localised exclusively on internal membranes. In contrast, 14% (n=3) of HEK293T cells transfected with HA-GABA_{B}-R2 showed surface immunoreactivity (Figure 7c). However, co-transfection of both Myc-GABA_{B}-R1b and HA-GABA_{B}-R2 led to the appearance of Myc-GABA_{B}-R1b on the surface of 20% (n=3) of cells analysed (Figure 7a), strongly suggesting that co-expression of GABA_{B}-R1b with GABA_{B}-R2 is necessary for surface expression of GABA_{B-}R1b.

### 7. Receptor Glycosylation studies

Endoglycosidases F and H can be used to differentiate between core and terminally glycosylated *N*-linked glycoproteins. Therefore, these enzymes were used to examine the glycosylation status of both GABA_{B}-R1 and GABA_{B}-R2 following expression in HEK293T cells. Membranes from transfected cells were treated with either endoglycosidase F or endoglycosidase H and expressed GABA_{B} receptors were characterised by immunoblotting to compare relative electrophoretic mobilities of the receptors (Figure 8). Cell membranes expressing either GABA_{B}-R1a or 1b produced distinct bands of *M*ᵣ 130 and 100K respectively (Figure 8, lanes 1 and 4) which following endoglycosidase F treatment, decreased in size to single immunoreactive species of *M*ᵣ 110 and 80K; representing GABA_{B}-R1a and GABA_{B}-R1b respectively (Figure 8, lanes 2 and 5). This shows that recombinant GABA_{B}-R1a and 1b are glycoproteins, in agreement with the observations of Kaupmann *et al.,* (1997). However, both GABA_{B}-R1a and 1 b splice variant forms were also sensitive to endoglycosidase H treatment, indicating that the expressed proteins are only core glycosylated (lanes 3 and 6) and lack terminal glycosylation. This observation, together with the FACS analysis, suggests that the proteins are immaturely glycosylated and retained on internal membranes. Significantly, when either GABA_{B}-R1a (lanes 7-9) or GABA_{B}-R1b (lanes 10-12) was co-expressed with HA-GABA_{B}-R2, a component of GABA_{B}-R1a or 1 b was resistant to endoglycosidase H digestion suggesting that when co-expressed with GABA_{B}-R2, a significant fraction of GABA_{B}-R1 is now a mature glycoprotein (lanes 9 and 12).

Similar studies with HA-GABA_{B}-R2 gave an immunoreactive species with an *M*ᵣ of 120 K (Figure 8, lanes 13, 16, 19) which was sensitive to endoglycosidase F (lanes 14, 17 and 20) but resistant to endoglycosidase H (lanes 15, 18 and 21) treatment, whether expressed alone or in combination with GABA_{B}-R1. Thus, these data indicate that expressed HA-GABA_{B}-R2 is a mature glycoprotein whose glycosylation status is not affected by co-expression with GABA_{B}-R1. Thus, heterodimerisation between GABA_{B}-R1 and GABA_{B}-R2, possibly in the Golgi complex, could be a prerequisite for maturation and transport of GABA_{B-}R1 to the plasma membrane.

### 8. Functional studies

To determine whether co-expression of GABA_{B}-R1 and GABA_{B}-R2 and its subsequent mature glycosylation and cell surface expression, generated a receptor complex able to functionally respond to GABA, we measured three types of signalling. We used transiently transfected HEK239T cells to examine firstly, activation of [³⁵S]GTPγS binding in membranes and secondly, inhibition of forskolin stimulated cAMP activation in whole cells. Thirdly we expressed GABA_{B}-R1 and GABA_{B}-R2 in *Xenopus* oocytes, expressing either the cystic fibrosis transmembrane regulator (CFTR) or inwardly rectifying K⁺ channels (GIRK and KATP) and examined activation of ion flux in response to agonist.

### i. [³⁵S]GTPγS binding

No GABA stimulated [³⁵S]GTPγS binding was observed in membranes prepared from cells transfected with either GABA_{B}-R1 or HA-GABA_{B}-R2 in combination with Gₒ₁α. However, co-expression of GABA_{B}-R1 and HA-GABA_{B}-R2 together with Gₒ₁α resulted in a robust stimulation of [³⁵S]GTPγS binding activity (Figure 9a). This was found to be concentration-dependent with similar EC₅₀ (mean, ± S.E.M., n = 3) values determined for membranes from cells transfected with HA-GABA_{B}-R2 and Gₒ₁α together with either GABA_{B}-R1a (9.5 ± 1.1 x 10⁻⁵M) or GABA_{B}-R1b (7.8 ± 0.4 x 10⁻⁵M) (Figure 9b). These values are equivalent to those of GABA-mediated stimulation of [³⁵S]GTPγS binding to rat brain membranes (5.9 ± 0.4 x 10⁻⁵M) (data not shown). We were concerned that an N-terminal HA epitope tag on GABA_{B}-R2 could alter receptor function and so we performed parallel studies in HEK293T cells, expressing untagged versions of GABA_{B}-R2 and GABA_{B}-R1 together with Gₒ₁α. Similar efficacies and potencies of GABA action were observed in membranes from these cells, as reported for the epitope tagged receptors (data not shown), clearly suggesting that the addition of these peptide sequences to the N-termini of GABA_{B}-R2 and GABA_{B-}R1 did not significantly alter receptor function. It is noteworthy that a measurable GABA-mediated elevation of [³⁵S]GTPγS binding activity was only observed upon co-expression of GABA_{B}-R1 and HA-GABA_{B}-R2 together with additional Gₒ₁α (Figure 10). The requirement for additional G protein is most likely due to relatively low levels of endogenously expressed G_{i/o} family G proteins, thus precluding a discernible GABA-mediated response upon GABA_{B}-R1 and GABA_{B}-R2 co-expression.

### ii cAMP inhibition

Similar results were obtained from HEK293T cells transiently transfected with GABA_{B}-R1 and GABA_{B}-R2, using inhibition of forskolin evoked cAMP as a readout. Once again, functional responses were only observed when both GABA_{B}-R1 and GABA_{B}-R2 were co-expressed (Figure 11).

### iii Xenopus oocytes

*Xenopus* oocytes can assay for three classes of G-protein:
1) Endogenous oocyte Ca²⁺-activated chloride conductance can assay for activation of G_{q} and a subsequent rise in intracellular calcium (Uezono et *al.,*1993)*.*
2) Cystic fibrosis transmembrane regulator (CFTR), which contains a cAMP-activated chloride channel, can assay for receptor activation via Gₛ or G_{i/o} (Uezono *et al.,* 1993; Wotta *et al.,* 1997).
3) G-protein regulated potassium channels GIRK1 (Kir 3.1; Kubo *et al*., 1993) and GIRK4 (or CIR, Kir 3.4, Kaprivinsky et *al*.,1995), injected in equal amounts to generate a heteromeric channel, can assay for activation of pertussis toxin sensitive G-proteins (Kovoor *et al*., 1997).

No functional responses to GABA or baclofen were seen when cloned GABA_{B-}R1a, GABA_{B}-R1b or GABA_{B}-R2 receptors were expressed in oocytes in combination with CFTR or GIRK1/4 (data not shown; see Figure 12b). When GABA_{B}-R1 and GABA_{B}-R2 were co-expressed with CFTR, several significant, robust responses were recorded following application of 100µM GABA (Figure 12a). Moreover, repeated application of GABA led to a progressive increase in the size of the CFTR response, suggesting that the functional response of the heterodimer is now sensitised to further challenge by agonist. This phenomenon has not been observed for other cloned receptors expressed in oocytes and may be related to the heterodimerisation or even oligomerisation of the GABA_{B} receptors. Finally, two other GABA_{B}-selective agonists, Baclofen and SKF97541 elicted similar functional responses through CFTR to that of GABA (Figure12a). In contrast, antagonists gave no response (data not shown).

Next, we examined the GABA_{B}-R1/GABA_{B}-R2 heterodimer with the G-protein regulated potassium channels GIRK1 and GIRK4 and once again found agonist dependant responses. Time course plots were examined for three individual oocytes expressing GABA_{B}-R2 alone (left panel), GABA_{B}-R1 plus GABA_{B}-R2 (middle panel) and the adenosine A1 receptor (as a positive control, right panel) (Figure 12b). In each case, switching from a low potassium physiological solution (ND96) to a high potassium extracellular solution (90mM K⁺) led to an inward shift in holding current, resulting from agonist-independent influx of potassium ions through the GIRK1/4 channel. No GABA response was seen in oocytes expressing GABA_{B}-R2 in isolation (Figure 12b, left panel) and similarly, GABA_{B}-R1a and GABA_{B}-R1b expressed alone also gave no response to GABA (data not shown). Significantly, a large GABA response was recorded in oocytes co-expressing GABA_{B}-R1 and GABA_{B}-R2 (Figure 12b, middle panel) of a similar magnitude to that of the adenosine A1 receptor in response to the agonist NECA (Figure 12b, right panel). Thus, once again co-expression of the two receptor subtypes elicits a functional agonist-dependant response, whereas expression of either subtype receptor alone does not. We also examined whether co-expression of the two receptors in oocytes could activate endogenous Ca²⁺-activated chloride conductance. No evidence for activation was seen (data not shown) suggesting that at least in oocytes, the GABA_{B-}R¹/GABA_{B}-R2 receptor complex does not signal through Gq. Finally, a current-voltage curve were constructed for an oocyte co-expressing GABA_{B}-R1 and GABA_{B}-R2 (Figure 13). This clearly demonstrates that GABA, bound to the GABA_{B} receptor, activates a large inwardly rectifying current consistent with activation of the GIRK potassium channel in a fully dose dependant manner.

### 9. Stoichiometric Studies on the Heterodimer

Since co-expression of GABA_{B}-R1 and GABA_{B}-R2 is necessary for a functional GABA_{B} receptor, we decided to investigate stoichiometric ratio between the two receptor subtypes *in vivo.* Relative levels of expression for both GABA_{B}-R1 and GABA_{B}-R2 were measured following transfection into HEK293T cells and compared to receptor function, as determined by GTPγS binding (Figure 14). Increasing amounts of HA-GABA_{B}-R1 (up to 1µg) plasmid were transfected into HEK293T cells along with a constant (1µg) amount of HA-GABA_{B}-R2. GABA caused stimulation of [³⁵S]GTPγS binding above basal levels in membranes extracted from these cells, which increased with increasing amount of transfected HA-GABA_{B}-R1 until binding reached a plateau when levels of HA-GABA_{B}-R1 were greater than 0.25µg (Figure 14a). Immunoblotting of the same membrane samples revealed equivalent levels of expression of HA-GABA_{B}-R1 and HA-GABA_{B}-R2 in membranes transfected with 0.25-0.5 µg of HA-GABA_{B}-R1 (Figure 14b). This corresponded to the plateau of GABA-mediated elevation of [³⁵S]GTPγS binding activity and therefore strongly suggests that GABA_{B}-R1 and GABA_{B}-R2 functionally interact in a 1:1 stoichiometric ratio.

### 10. Competition Binding Studies

Finally, we determined whether the observed functional responses were due to a high affinity GABA_{B} receptor, composed of a heterodimer of the two receptors. HEK293T cells were transfected with either 1µg HA-GABA_{B}-R1b and HA-GABA_{B}-R2 individually or with increasing amounts (up to 1µg) of HA-GABA_{B-}R1 b and a fixed amount (1µg) of HA-GABA_{B}-R2 together with Gₒ₁α. Competition binding assays were then performed upon purified membranes. Expression of HA-GABA_{B}-R1b alone produced high levels of specific binding of [³H]-CGP54626 (Bittiger *et al.,* 1992), a structural analogue of [¹²⁵I]-CGP64213 and the antagonist originally used to expression clone GABA_{B}-R1 (Kaupmann *et al.,* 1997). However, as previously reported for [¹²⁵I]-CGP64213, GABA inhibition curves were significantly shifted to the right compared with binding to rat brain membranes (Figure 15), giving approximately 22-fold lower IC₅₀ than rat brain binding. Significantly, co-expression of equivalent amounts of HA-GABA_{B}-R1b and HA-GABA_{B}-R2 protein revealed high levels of specific binding. In a control experiment using untagged receptors similar values were obtained (data not shown). Achievement of a 1:1 stoichiometric ratio of expression of HA-GABA_{B-}R1 b and HA-GABA_{B}-R2 led to agonist inhibition curves similar to those obtained in rat brain membranes (IC₅₀ ± 95% confidence intervals for 1µg HA-GABA_{B}-R2 / 0.25µg HA-GABA_{B}-R1b = 2.29µM (1.48-3.55µM) and for rat brain = 1.04µM (0.69-1.58µM). Such comparable levels of receptor expression were also shown to permit optimal agonist activation in the GTPγS assay (see Figure 14). Alteration of receptor ratio from 1:1, such that GABA_{B}-R1b was the most prevalent receptor, led to reduced agonist affinity, presumably due to binding at non-dimerised and immaturely glycosylated GABA_{B}-R1b receptors (Figure 15). In addition, despite its apparent cell surface expression, we were unable to detect any [³H]-CGP54626 specific binding to HEK293T cells transiently transfected with HA-GABA_{B}-R2 alone (data not shown). We conclude that heterodimerisation of the GABA_{B}-R1 and GABA_{B}-R2 subtypes are necessary to generate a high affinity GABA_{B} receptor. There are a number of possible explanations for the change in GABA affinity following co-expression of the two receptor subtypes. Appearance of the GABA_{B} receptor complex at the cell surface would be expected to allow G protein coupling of the receptor which would increase agonist affinity. However, in previous studies is has been shown that the lack of G protein coupling alone cannot account for the difference in agonist affinity between rat brain receptors and GABA_{B}-R1 (Kaupmann *et al.,* 1997). Furthermore, we have noted that [³H]-CGP54626 appears to primarily bind the low affinity state of the receptor, even in rat brain membranes, as demonstrated by the fact that GTPγS is unable to shift agonist inhibition curves and actually increases the level of ³H-CGP54626 specific binding (data not shown). Therefore, a more likely explanation for the change in GABA affinity following co-expression of the two GABA_{B} receptors is that heterodimerisation together with the mature glycosylation state of the protein, produces a binding site conformation with an inherent higher affinity.

### Discussion

Functional GABA_{B} receptors within the CNS comprise a cell surface heterodimer of two distinct 7-transmembrane receptor subunits, GABA_{B}-R1 and GABA_{B}-R2 in a 1:1 stoichiometric ratio. *in vivo,* GABA_{B} receptors may exist simply as heterodimers or form even larger multimeric complexes of many heterodimers. Formation of the heterodimer via the coiled-coil domains within the receptor C-terminal tails appears to be a pre-requisite for transport and full glycosylation of GABA_{B}-R1, as well as for the generation of a high affinity GABA_{B} receptor at the cell surface. Using.this information, we have been able to reproduce GABA_{B} sites in both mammalian HEK293T cells as well as in oocytes, using several functional readouts such as activation of ion flux through CFTR or GIRK in oocytes, or inhibition of adenylyl cyclase in HEK293T cells. Indeed the lack of functional responses in cells expressing GABA_{B}-R1 alone and the need for expression of a second 7TM receptor explains why many groups have encountered extreme difficulty in expression cloning a GABA_{B} receptor via conventional means. We believe this is the first report of receptor heterodimerisation as an obligate requirement to generate a high affinity, fully functional receptor in recombinant systems, which is fully equivalent to that of endogenous tissues.

Dimerisation has been reported for other receptor families, such as the opioid family as a part of their desensitisation process, the β2-adrenergic receptor, where homodimers may play a role in signalling, and the metabotropic glutamate receptors (mGluRs, Hebert *et al.,* 1996; Romano *et al.,* 1996; Cvejic *et al.,* 1997, Hebert and Bouvier, 1998). Significantly, dimerisation in these receptor families does not appear to be an absolute requirement for functional coupling in recombinant systems. In the case of the mGluRs, which are a closely related receptor family to GABA_{B} (Kaupmann *et al.,* 1997), homodimerisation is mediated through disulphide bridges between the N-terminal extracellular domains rather than a C-terminal coiled-coil. Indeed, heterodimerisation between two 7-transmembrane receptors, leading to both trafficking and mature glycosylation of the proteins to yield a functional receptor is unprecedented and is unique in the GPCR field. Certainly, mGluRs have not been found to form heterodimers (Romano *et al.,* 1996) and the fact that two such closely related receptors families have evolved such different mechanisms of dimer formation suggests that this is a fundamentally important process for receptor function.

*In vivo,* pharmacotogical evidence suggests that there are many different GABA_{B} receptor subtypes; both within the CNS as well as in peripheral tissues. How are such pharmacological subtypes of GABA_{B} receptors formed? Only GABA_{B}-R1 and GABA_{B}-R2 have. been identified as separate genes to date and database trawling has not identified any further receptors homologous to known GABA_{B} receptors. This does not exclude the possibility that more, as yet unrecognised GABA_{B} receptors do exist. Differences in distribution exist for the two GABA_{B} receptors, for example GABA_{B}-R2 is specifically expressed in the CNS whereas GABA_{B}-R1 is expressed in both central and peripheral sites. These differences in distribution clearly add further complexity leading to the pharmacologically distinct receptor subtypes. Moreover, the genes encoding the GABA_{B} receptors may be differentially spliced. GABA_{B}-R1 encodes three N-terminal splice variants and yet more may remain to be detected. Interestingly, these splice variants have alterations in their N-terminal, extracellular domain, the region involved in GABA binding (Takahashi *et al.,* 1993, O'Hara *et al.,* 1993) and encode either two (GABA_{B}-R1a), one (GABA_{B}-R1c) or no (GABA_{B}-R1b) sushi domains. Given that the sushi domains mediate cell-cell protein-protein contact, the differences in these three splice variants may account for yet more of the pharmacologically defined GABA_{B} receptor subtypes. To date, we have not detected any splice variants to GABA_{B}-R2. Furthermore there are significant differences in the distribution of the individual splice variants suggesting that they may serve different functions within the CNS. For instance, GABA_{B}-R1a splice variant is reported as presynaptic within the brain (Bettler *et al.,* 1998) and therefore may define presynaptic GABA_{B} autoreceptors. It seems likely that these splice variants of GABA_{B}-R1 may account for at least some of the pharmacologically defined subtypes. Finally, with this novel observation of obligate receptor heterodimerisation, a further level of complexity has been added since functional GABA_{B} binding sites require a heterodimerisation partner.
Now the molecular nature of the GABA_{B} receptor is more fully understood, recombinant systems can be established for high throughput screening for compounds against individual pharmacologically defined GABA_{B} sites. By these means, compounds with greater specificity and with fewer unwanted side effects can be discovered. For this, GABA_{B}-R1 and GABA_{B}-R2 (including all spice variants, and any fragments of the receptor) should be co-expressed either stably or transiently in suitable host cells. Suitable host cells include higher eukaryotic cell lines, such as mammalian cells, insect cells, lower eukaryotic cells, such as yeast or prokaryotic cells such as a bacterial cells. Screening assays with these recombinant cell lines could involve the use of radioligand binding to the dimer or individual subunits within the dimer. The activity profile in a binding assay to the dimer is likely to be different from the activity of compounds assayed using binding assays to GABA_{B}-R1 alone due to alterations in the glycosylation status and the conformation of the receptor as a result of co-expressing GABA_{B}-R1 or GABA_{B}-R2. Functional assays, which measure events downstream of receptor activation, can also be used for screening compounds. Such assays include [³⁵S]-GTPγS binding to membranes isolated from cells expressing the dimer; activation or inhibition of ion channels using electrophysiological recording or ion flux assays; mobilisation of intracellular calcium; modulation of cAMP levels; activation or inhibition of MAP kinase pathways or alterations in the activity of transcription factors with the use of reporter genes. Further to this, secondary screens can be established in a similar manner, using different heterodimer combinations to exclude unwanted activity and thereby establish subtype selective GABA_{B} compounds.

In addition, any approach targetting the disruption or enhancemant of dimer formation of the GABA_{B} heterodimer could represent a novel therapeutic approach with which to target GABA_{B} receptors. Such strategies could include peptides or proteins physically associated with the coiled-coil domain or indeed, any other interacting regions of the dimer. Small molecules could also be identified which act at the points of contact formed by interaction of the components of the dimer. These may either promote or enhance the receptor function. Finally, antibodies could be made which specifically recognise epitopes on the dimer, as opposed to the monomer subunits. These could be used as tools to further elucidate the function of GABA_{B} receptors in disease or as therapeutic agents in their own right.

### Methods

### DNA Manipulation

Standard molecular biology protocols were used throughout (Sambrook *et al.,* 1989) and all bacterial manipulations used Escherichia coli XL-1Blue (Stratagene) according to the manufacturers instructions. Standard PCR conditions were used throughout, unless otherwise stated. PCR reaction mixture contained 10-50ng of target DNA, 1 pmol of each primer; 200µM dNTPs and 2.5U of either Taq polymerase (Perkin-Elmer) or Pful polymerase (Stratagene) with the appropriate buffer as supplied by the manufacturer. Cycling parameters were 1cycle 95°C 2mins; 25 cycles 95°C 45secs 55°C 45secs 72°C 1min; 1cycle 72°C 10mins. All PCR were carried out using either a Perkin Elmer 9600 PCR machine or a Robocycler Gradient 96 (Stratagene) PCR machine.

### GABA_{B}-R1 - Cloning of Human homologues and splice variants

Several human EST's (X90542; X90543; D80024; AA348199; T06711; T07518 and AA38224) were identified as homologous to the rat GABA_{B}-R1a and GABA_{B}-R1b sequences (Y10369; Y10370). The ESTs were aligned and the predicted open reading frame was amplified by RT-PCR from human brain cerebellum polyA⁺ RNA (Clontech) using the Superscript Preamplification System (Life Technologies). The 3' end of the receptor (1545-2538bp; GABA_{B-}R1 b) was amplified using primers 5'-GCGACTGCTGTGGGCTGCTTACT GGC-3 and 5'-GCGAATTCCCTGTCCTCCCTCACCCTACCC-3'. The central section (277-1737bp of GABA_{B}-R1b) was amplified using 5'-CCGAGCTCAAGCTCATCCACCACG-3' and 5'-TCTTCCTCCACTCCTTCTTTTCTT-3'. PCR products were subcloned into pCR-Script SK(+) (PCR-script Amp cloning kit; Stratagene). Error free PCR product were assembled in a three-way *Bst*EII, *Sac*I and *Eco*RI ligation and subcloned into pBluescript SK (-) (Stratagene).

The N-termini of the splice variants were generated using RACE (rapid amplification of cDNA ends) PCR with the Marathon cDNA amplification kit against Marathon-Ready human cerebellum cDNA (Clontech). RACE PCR was primed from a conserved sequence within GABA_{B}-R1 using primer 5'-TGAGCTGGAGCCATAGGAAAGCACAAT-3' to generate a 700bp product. This further PCR amplified using the AP2 primer (Marathon) and a second internal GABA_{B}-R1 primer 5'-GATCTTGATAGGGTCGTTGTAGAGCA-3'. The resulting 600bp product was subcloned using the Zero blunt PCR cloning kit (Invitrogen). Sequence information achieved from this RACE PCR was used to clone the N-terminus of the GABA_{B}-R1b splice variant, using primers 5'-GCTCCTAACGCTCCCCAACA-3' and 5'-GGCCTGGATCACACTTGCTG-3' into pCR-Script SK (+)(Stratagene). Human GABA_{B}-R1a 5' sequences were retrieved from Incyte database EST's (1005101;3289832) and used to design primers 5'-CCCAACGCCACCTCAGAAG-3' and 5'-CCGCTCATGGGAAACAGTG C-3'. PCR on cerebellum cDNA and KELLY neuroblastoma cell line cDNA produced two discreet bands at 300bp and 400bp, which were cloned into pCR-Script SK (+) (Stratagene). Sequencing revealed that the 400bp product encoded some of the Human GABA_{B}R1a 5' sequences and the 300bp product encoded the novel splice variant, GABA_{B}-R1c. Next, primer, 5'-CCCCGGCACACATACTCAATCTCATAG-3' was designed to RACE PCR the missing ~225bp of GABA_{B}-R1a. A 250bp product was obtained and reamplified using primer 5'-CCGGTACCTGATGCCCCCTTCC-3' with primer AP2 (Marathon). A ~250bp band was once again generated, subcloned into pCR-Script SK (+) and when sequenced, encoded the 5' end of GABA_{B}-R1a. Next, clones spanning both the conserved receptor sequence and the %' ends of the splice variants GABA_{B}-R1a and GABA_{B}-R1c were generated. Primer 5'-CGAGATGTTGCTGCTGCTGCTA-3', priming from the start codon and the reverse RACE primer generated a predicted ~800bp band and this was subcloned into pCR-Script SK(+). Now, full-length GABA_{B}-R1a, GABA_{B}-R1b and GABA_{B}-R1c clones can be assembled in pcDNA3.1 (-) (Invitrogen). For GABA_{B-}R1 b, 5' sequences, restricted Notl /Sacl, and the conserved region of the receptor, cut EcoRI/SacI were both co-ligated into pcDNA3.1 (-), restricted Notl /EcoRl. Likewise, the GABA_{B}-R1a and GABA_{B}-R1c 5' fragments were subcloned Xhol /Sacl with the EcoRl/Sacl conserved fragment and co-ligated into pcDNA3.1(-) , cut XhoI/EcoRI to reconstitute full length clones.

### Tagging of GABA_{B}-R1b

GABA_{B}-R1b was tagged with either myc or HA epitopes. PCR primers 5'-TAGGATCCCACTCCCCCCATCCC-3' and 5'-CCAGCGTGGAGACAGAGCTG-3' were used to amplify a region immediately following the proposed signal sequence (position 88) to approx. 20bp downstream of a unique Pstl site at position 389 of the coding sequence, creating a unique 5' in-frame BamHl site. This fragment was cloned ,BamHl/Pstl , into a vector containing the CD97 signal sequence, the myc epitope and an in-frame BamHl site. This construct also contains a Notl site 5' to the CD97 signal sequence and an EcoRl. site downstream of the Pstl site. GABA_{B}-R1b sequences downstream to the Pstl site and upto an external EcoRl site were subcloned from full length receptor into the vector described above likewise cut with Pstl/EcoRl, to assemble full length tagged GABA_{B}-R1b. CD97 signal sequence, myc epitope and GABA_{B}-R1b coding sequence were subcloned, NotI/EcoRI, into pCDNA3.1 (-) (Invitrogen). HA epitope was added to GABA_{B} R1b by co-ligation of the 5' BamHI/PstI and 3' PstI/EcoRI fragments into pCIN6 cut with BamHI/EcoRI. This vector contains a T8 signal sequence and 12CA5 HA epitope immediately preceding an in-frame BamHI site.

### Cloning of GABA_{B}-R2, the novel GABA_{B} receptor subtype

EST clones (H14151, R76089, R80651, AA324303, T07621, Z43654) were identified with approximately 50% nucleotide identity to GABA_{B}-R1. PCR revealed that H14151 contained a 1.5Kb insert and encoded sufficient sequence for a substantial portion the novel GABA_{B} receptor. PCR between the 3' end of H14151 and the 5' end of AA324303, using a cerebellum cDNA library as template, produced a -700bp product, which when cloned into the T-vector (TA cloning kit, Invitrogen) and sequenced, revealed that T07621 overlaps within AA324303. Also, Z43654 as well as genomic DNA fragments R76089 and R80651 were found to overlap AA324303 and together provided sequence data for the 3' end of the GABA_{B} subtype receptor. Further sequencing of H14151 provided the full sequence for the novel receptor subtype. However, because of ambiguities in the position of the stop codon in Z43654/R80448/R80651, Incyte clones 662098 and 090041, which overlap this region, were sequenced. The stop codon was identified and sequence for GABA_{B}-R2 was confirmed as within H14151 (5' end) and 662098 (3' end). 5' sequences of GABA_{B}-R2 were PCR generated using primers 5'-ATGGCTTCCCCGCGGAG-3' to provide the start codon of the receptor and primer 5'-GAACAGGCGTGGTTGCAG-3', priming beyond a unique EagI site. The expected ~250bp product was cloned into pCRSCRIPT and sequenced. Full length receptor was then assembled with a three way ligation between H14151, cut with ApaLl /Eagl; 662098, cut with ApabI/NotI and pCRSCRIPT- GABA_{B}-R2-5' PCR product, restricted by EagI.Full length GABA_{B}-R2 was removed from the pCRSCRIPT vector using EcoRI/NotI and ligated into pcDNA3 (Invitrogen) for expression studies.

HA-epitope tagged GABA_{B}-R2 was constructed in pCIN6,.A linker was constructed encoding amino acids between the GABA_{B}-R2 signal sequence and the unique EagI site. The linker was cloned into pUC18 (EcoRI /HindIII) followed by full length GABAB-R2, from pCRSCRIPT as an EagI/NotI fragment. Finally, the modified GABA_{B}-R2 was cloned into pCIN6 as a Xhol fragment.

### Distribution Studies

Blots were hybridized overnight at 65°C according to the manufacturers' instructions with radioactively randomly primed cDNA probes using ExpressHyb Hybridization solution. Probe for GABA_{B}-R1, corresponding to residues 1129-1618 of the GABA_{B}-R1b coding sequence was PCR amplified using primers 5'-CGCCTGGAGGACTTCAACTACAA-3' and 5'-TCCTCCCAATGTGGTAACCATCG-3' against GABA_{B}-R1b DNA as template.

GABA_{B}-R2 cDNA probe, corresponding to residues 1397-1800, was amplified by PCR using primers 5'-ACAAGACCATCATCCTGGA-3' and 5'-GATCACAAGCAGTTTCTGGTC-3' with GABA_{B}-R2 DNA as template. DNA fragments were labelled with ³²P-α-dCTP using a Rediprime DNA labelling system (Amersham). Probes were labelled to a specific activity of >10⁹ cpm/µg and were used at a concentration of approximately 5ng/ml hybridization solution. Following hybridization, blots were washed with 2xSSC/1% SDS at 65°C, and 0.1xSSC/0.5% SDS at 55°C (20xSSC is 3M NaCl/0.3M Na₃Citrate.2H₂0 pH7.0) and were exposed to X-ray film.

### Yeast Two Hybrid Studies

*Saccharomyces cerevisiae* Y190 [ *MATa, gal4 gal80, ade2-101, his3, trp1-901, ura3-52, leu2-3, 112, URA3::GAL1-lacZ, LYS2:: GAL1-HIS3, cyh*^{*R*}] was used for all described yeast two hybrid work (Harper *et al.,* 1993, Clontech Laboratories, 1996). GAL4 binding-domain (GAL4_{BD}) fusion vectors were constructed in either pYTH9 (Fuller et al., 1998) or pYTH16, an episomal version of pYTH9. All GAL4 . activation-domain-fusions were made in pACT2 (Clontech Laboratories, 1998) All yeast manipulations were carried out using standard yeast media (Sherman, 1991). Human Brain MATCHMAKER library (HL4004AH) in pACT2 was purchased from Clontech Laboratories and amplified according to the manufacturers' instructions. The GABA_{B}-R1 C-terminal domain was amplified from a full length clone, using primers 5'-GTTGTCCCCATGGTGCCCAAGATGCGCA GGCTGATCACC-3' and 5'-GTCCTGCGGCCGCGGATCCTCACTTATAAAGCAAATGCACT CG-3'. PCR product was size-fractionated on 0.8% agarose gel, purified and force-cloned Ncol/Notl into pYTH9 and subsequently into pACT2. The GABA_{B-}R2 C-terminal domain was similarly generated with primers 5'-CTCTGCCCCATGGCCGTGCCGAAGCTCATCACCCTGA GAACAAACCC-3' and 5'-GGCCCAGGGCGGCCGCACTTACAGGCCCGAGACCATGACTC GGAAGGAGGG-3' and subcloned into pYTH9, pYTH16 and pACT2. All cloned PCR products were sequenced and confirmed as error free.

The GAL4_{BD}-GABA_{B}-R1 C-terminus fusion in pYTH9 was stably integrated into the trp1 locus of Y190 by targetted homologous recombination. Yeast expressing GAL4_{BD}-GABA_{B}-R1 C-terminus were selected and transformed with Human brain cDNA library under leucine selection, using a high efficiency Lithium acetate transformation protocol (Clontech Laboratories, 1998). Sufficient independent cDNAs were transformed to give a three fold representation of the library. Interacting clones were selected by growth under 20mM 3-amino-1,2,4-triazole (Sigma) selection, followed by production of β-galactosidase, as determined by a freeze-fracture assay (Clontech Laboratories, 1998). Plasmid DNA was recovered from yeast cells following digestion of the cell wall by 400µg/ml Zymolase 100T (ICN Biochemicals) in 250µl 1.2M Sorbitol; 0.1M potassium phosphate buffer (pH 7.4) at 37°for 2 h. Plasmid DNA was extracted by standard Qiagen alkaline lysis miniprep as per manufacturers' instructions and transformed into Ultracompetent XL-2Blue cells (Stratagene). Plasmid DNA was sequenced using primer 5'-CAGGGATGTTTAATACCACTACAATGG-3' using automated ABI sequencing and resulting sequences were blasted against the databases.

Yeast Y190 was transformed with pYTH16 and pACT2 expressing GABA_{B}-R1 C-terminal domain and the GABA_{B}-R2 C-terminal domain in all combinations, as well as against empty vectors. Transformants were grown in liquid media to mid-logarithmic phase and approximately 1.5ml harvested. β-galactosidase activity was quantified using substrate o-nitrophenyl β-D-galactopyranoside (ONPG; Sigma) using a liquid nitrogen freeze fracture regime essentially as described by Harshman *et al.,* (1988).

### Two-microelectrode voltage-clamp in Xenopus oocytes

Adult female *Xenopus laevis* (Blades Biologicals) were anaesthetised using 0.2% tricaine (3-aminobenzoic acid ethyl ester), killed and the ovaries rapidly removed. Oocytes were de-folliculated by collagenase digestion (Sigma type I, 1.5 mg ml⁻¹) in divalent cation-free OR2 solution (82.5mM NaCl, 2.5mM KCI, 1.2mM NaH₂PO₄, 5mM HEPES; pH 7.5 at 25°C). Single stage V and VI oocytes were transferred to ND96 solution (96mM NaCl, 2mM KCI, 1mM MgCl₂, 1.8mM CaCl₂, 5mM HEPES; pH 7.5 at 25°C) which contained 50µg ml⁻¹ gentamycin and stored at 18°C.

GABA_{B}-R1a, GABA_{B}-R1b (both in pcDNA3.1rev, Invitrogen), GABA_{B}-R2, GIRK1, GIRK4 (in pcDNA3) and cystic fibrosis transmembrane regulator (CFTR; in pBluescript, Stratagene) were linearised and transcribed to RNA using T7 or T3 polymerase (Promega Wizard kit). m'G(5')pp(5')GTP capped cRNA was injected into oocytes (20-50nl of 1µgµl⁻¹ RNA per oocyte) and whole-cell currents were recorded using two-microelectrode voltage-clamp (Geneclamp amplifier, Axon instruments Inc.) 3 to 7 days post-RNA injection. Microelectrodes had a resistance of 0.5 to 2MΩ when filled with 3M KCl. In all experiments oocytes were voltage-clamped at a holding potential of -60mV in ND96 solution (superfused at 2ml per min.) and agonists were applied by addition to this extracellular solution. In GIRK experiments the extracellular solution was changed to a high potassium solution prior to agonist application, to facilitate the recording of inward potassium currents. Current-voltage curves were constructed by applying 200ms voltage-clamp pulses from the holding potential of-60mV to test potentials between -100mV and +50mV.

### Mammalian Cell culture and transfections

HEK293T cells (HEK293 cells stably expressing the SV40 large T-antigen) were maintained in DMEM containing 10 % (v/v) foetal calf serum and 2 mM glutamine. Cells were seeded in 60 mm culture dishes and grown to 60-80 % confluency (18-24 h) prior to transfection with pCDNA3 containing the relevant DNA species using Lipofectamine reagent. For transfection, 3 µg of DNA was mixed with 10 µl of Lipofectamine in 0.2 ml of Opti-MEM (Life Technologies Inc.) and was incubated at room temperature for 30 min prior to the addition of 1.6 ml of Opti-MEM. Cells were exposed to the Lipofectamine/DNA mixture for 5 h and 2 ml of 20 % (v/v) newborn calf serum in DMEM was then added. Cells were harvested 48-72 h after transfection.

### Preparation of membranes

Plasma membrane-containing P2 particulate fractions were prepared from cell pastes frozen at -80°C after harvest. All procedures were carried out at 4°C. Cell pellets were resuspended in 1 ml of 10 mM Tris-HCl and 0.1 mM EDTA, pH 7.5 (buffer A) and by homogenisation for 20 s with a polytron homogeniser followed by passage (5 times) through a 25-guage needle. Cell lysates were centrifuged at 1,000 *g* for 10 min in a microcentrifuge to pellet the nuclei and unbroken cells and P2 particulate fractions were recovered by microcentrifugation at 16,000 *g* for 30 min. P2 particulate fractions were resuspended in buffer A and stored at -80°C until required. Protein concentrations were determined using the bicinchoninic acid (BCA) procedure (Smith *et al.,* 1985) using BSA as a standard.

### High affinity [³⁵S]GTPγS binding

Assays were performed in 96-well format using a method modified from Wieland and Jakobs, 1994. Membranes (10 mg per point) were diluted to 0.083 mg/ml in assay buffer (20 mM HEPES, 100 mM NaCl, 10 mM MgCl₂, pH7.4) supplemented with saponin (10 mg/l) and pre-incubated with 40 mM GDP. Various concentrations of GABA were added, followed by [³⁵S]GTPgS (1170 Ci/mmol, Amersham) at 0.3 nM (total vol. of 100 ml) and binding was allowed to proceed at room temperature for 30 min: Non-specfic binding was determined by the inclusion of 0.6 mM GTP. Wheatgerm agglutinin SPA beads (Amersham) (0.5 mg) in 25 ml assay buffer were added and the whole was incubated at room temperature for 30 min with agitation. Plates were centrifuged at 1500 g for 5 min and bound.[³⁵S]GTPgS was determined by scintillation counting on a Wallac 1450 microbeta Trilux scintillation counter.

### Measurement of cAMP levels

24 hours following transfection, each 60 mm dish of HEK293T cells was split into 36 wells of a 96-well plate and the cells were allowed to reattach overnight. Cells were washed with PBS and pre-incubated in DMEM medium containing 300 µM IBMX for 30 minutes at 37°C. Forskolin (50 µM) and varying concentrations of GABA were added and cells incubated for a further 30 min prior to cAMP extraction with 0.1M HCl for 1h at 4°C. Assays were neutralised with 0.1 M KHCO₃ and cAMP levels determined using scintillation proximity assays (Biotrak Kit, Amersham).

### Flow Cytometric Analysis

HEK293T cells were transiently transfected with cDNA as described. 48-72h following transfection, cells were recovered and washed twice in PBS supplemented with 0.1 % (w/v) NaN₃ and 2.5 % (v/v) foetal calf serum. Cells were resuspended in buffer and incubated with primary antibodies 9E10 (c-Myc) or 12CA5 (HA) for 15 min at room temperature. Following three further washes with PBS, cells were incubated with secondary antibody (sheep anti-mouse Fab₂ coupled with fluorescein isothiocyanate (FITC)) diluted 1:30 for 15 min at room temperature. For permeabilised cells, a Fix and Perm kit (Caltag) was used. Cell analysis was performed on a Coulter Elite flow-cytometer set up to detect FITC fluoresence. 30,000 cells were analysed for each sample.

### Immunological studies

Antiserum 501 was raised against a synthetic peptide corresponding to the C-terminal 15 amino acids of the GABA_{B}-R1 receptor and was produced in a sheep, using a conjugate of this peptide and keyhole limpet hemocyanin (Calbiochem) as antigen. Membrane samples ,30-60 µg) were resolved by SDS-PAGE using 10 % (w/v) acrylamide. Following electrophoresis, proteins were subsequently transferred to nitrocellulose (Hybond ECL, Amersham), probed with antiserum 501 at 1:1000 dilution and visualised by enhanced chemiluminescence (ECL, Amersham). Epitope tags were visualised by immunoblotting with anti-Myc (9E10; 1:100 dilution) or anti-HA (12CA5; 1:500) monoclonal antibodies.

### Deglycosylation

Enzymatic removal of asparagine-linked (N-linked) carbohydrate moieties with endoglycosidases F and H was performed essentially according to manufacturers' instructions (Boehringer Mannheim) using 50 µg of membrane protein per enzyme reaction. GABA_{B} receptor glycosylation status was studied following SDS-PAGE/immunoblotting of samples.

### Immunoprecipitation procedures

Transiently transfected HEK293T cells were harvested as described above from 60mm culture dishes. Cells from each dish were resuspended in 1 ml of 50 mM Tris-HCl, 150 mM NaCl, 1% (v/v) Nonidet^{®} P40, 0.5 % (w/v) sodium deoxycholate, pH 7.5 (lysis buffer) supplemented with Complete^{™} protease inhibitor cocktail tablets (1 tablet/25 ml) (Boehringer Mannheim). Cell lysis and membrane protein solubilisation was achieved by homogenisation for 20 seconds with a polytron homogeniser, followed by gentle mixing for 30 min at 4°C. Insoluble debris was removed by microcentrifugation at 16,000 *g* for 15 min at 4°C and the supernatant was pre-cleared by incubating with 50 µl of Protein A-agarose (Boehringer Mannheim) for 3 h at 4°C on a helical wheel to reduce non-specific background. Solubilised supernatant was divided into 2 x 500 µl aliquots and 20 µl of either HA or Myc antisera was added to each. Immunoprecipitation was allowed to proceed for 1 h at 4°C on a helical wheel prior to the addition of 50 µl of Protein A-agarose suspension. Capture of immune complexes was progressed overnight at 4°C on a helical wheel. Complexes were collected by microcentrifugation 12,000g for 1 min at 4°C and supernatant was discarded. Beads were washed by gentle resuspension and agitation sequentially in 1 ml of 50 mM Tris-HCl, pH 7.5, 500 mM NaCl, 0.1 % (v/v) Nonidet^{®} P40 and 0.05 % (w/v) sodium deoxycholate followed by 1 ml of 50 mM Tris-HCl, pH 7.5, 0.1 % (v/v) Nonidet^{®} P40 and 0.05 % (w/v) sodium deoxycholate. Immunoprecipitated proteins were released from Protein A-agarose by incubation in 30 µl of SDS-PAGE sample buffer at 70°C for 10 min and analysed by SDS-PAGE followed by immunoblotting.

### Binding Assays

Competition binding assays were performed in 50mM Tris HCl buffer (pH7.4) containing 40µM isoguvacine (Tocris Cookson) to block rat brain GABA_{A} binding sites. P2 membrane preparations were made from HEK293T cells transfected using conditions described above. Increasing concentrations of GABA were added to displace the antagonist [3H]-CGP 54626 (Tocris Cookson, 40Ci/mmol). Assay conditions were 0.4-0.6nM [³H]-CGP54626, incubated with 50µg/tube crude rat brain 'mitochondrial' fractions or 25µg/tube HEK293T P2 membranes at room temperature for 20 minutes. The total volume per tube was 0.5ml and non specific binding was determined using 1 mM GABA. Bound ligand was recovered using a Brandel 48 well harvester onto GF/B filters (Whatman) and measured by liquid scintillation using a Beckman LS6500 counter.

### References:

Bettler, B., Kaupmann, K and Bowery, N. Curr Opin in Neurobiol **8:** 345-350 (1998)
Bittiger, H., Froestl, W., Gentsch, C., Jaekel., J., Mickel, S.J., Mondori, C., Olpe, H.R., Schmuz, M. (1996) in *GABA: receptors, transporters and metabolism* Ed: C. Tanaka and N.G. Bowery. Birkhauser Verlag Basel Switzerland.
Bittiger, H., Reymann, N., Froestl, W. & Mickel, S. J. *Pharmacology Communications* **2** :1-2: 23 (1992)
Bowery, N.G., Hudson, A.L., Price, G.W. *Neuroscience* 20: 365-383 (1987)
Chou, K.C. and Heinrikson, R.L. J. *Protein Chem****-*16*:*** 765-773 (1997)
Clontech Laboratories. CLONTECH MATCHMAKER^{™} GAL4 Two Hybrid system User Manual, Protocol PT3061-1. Palo Alto, CA. (1996)
Cvejic, S. and Devi, L.A. *J. Biol Chem* **272:** 26959-26964 (1997)
Fuller, K.J., Morse, M.A., White, J.H.M., Dowell, S.J. and Sims, M.J. *BioTechniques* **25**: 85-92 (1998)
Gemignani, A., Paudice, P., Bonanno, G., Raiteri, M. *Mol Pharmacol* **46:** 558-562 (1994)
Harayama, N., Shibuya, I., Tanaka, K., Kabashima, N., Ueta, Y., Yamashita, H. *J. Physiol (Lond)* **509:** 371-383 (1998)
Harper, J.W., Adami, G.R., Wei, N. Keyomarsi, K. and Elledge, S.J. Cell **75:** 805-816 (1993)
Harshmann, K.D., Moye-Rowley, W.S. and Parker, C.S. *Cell* **53:** 321-330 (1988) Hebert, T.E. and Bouvier, M. *Biochem. Cell. Biol.* **76:** 1-11 (1998)
Hebert, T.E., Moffett, S., Morello, J.P., Loisel, T.P., Bichet, D.G., Barret, C., and Bouvier, M. *J. Biol Chem* **271:** 16384-16392 (1997)
Hill, D.R. and Bowery, N.G. *Nature (Lond)* **290:** 149-152 (1981)
Kaprivinsky, G., Gordon, E.A., Wickman, K., Velimirovic, B., Kaprivinsky, L. and Clapham, D.E. *Nature (Lond)* **374:** 135-141 (1995)
Kaupmann, K., Huggel, K., Heid, J., Flor, P.J., Bischoff, S., Mickel., S.J., McMaster, G., Angst; C., Bittiger, H., Froestl, W. and Bettler, B. *Nature(Lond)* **386**, 239-246 (1997)
Kerr, D.I., Humeniuk, R.E., and Ong, J. *Eur. J. Pharmacol.* **262**: 189-192 (1994)
Kerr, D.I. and Ong, J. *Pharmacol. Ther.* **67**: 187-246 (1995)
Kerr, D.I. and Ong. J. *DDT* **1** : 371-380 (1996)
Kobrinsky, E.M., Pearson, H.A. and Dolphin, A.C. *Neuroscience* **58:** 539-552 (1994).
Kovoor, A., Nappey, V., Kieffer, B.L. and Chavkin, C. J. *Biol. Chem.* **272:** 27605-27611. (1997)
Kubo, Y., Reuveny, E., Slesinger, P.A., Jan, Y.H. and Jan, L.Y. *Nature (Lond)* **364**: 802-806. (1993)
Lovinger, D.M., Harrison, N.L. and Lambert, N.A. *Eur. J. Pharmacol.* **211:** 337-341 (1992)
Lupas, A. *Trends in Biol. Sci.* **21:** 375-382 (1996)
Malcangio, M. and Bowery, N.G. *Clin Neuropharmacol* **18:** 285-305 (1995)
McLatchie, L. M., Fraser N.J., Main, M.J. Wise A., Brown, J., Thompson, N., Solari, R., Lee, M.G and Foord, S.M. *Nature (Lond)* **393**: 333-339 (1998)
Menon-Johansson, A.S., Berrow, N. and Dolphin, A.C. *Pflugers Arch* **425**: 335-343 (1993)
Ohmori, Y., Hirouchi, M., Taguchim J. and Kuriyama, K. J. *Neurochem* **54**: 80-85 (1990)
Ong, J., Kerr, D.I., Doolette, D.J., Duke, R.K., Mewett, K.N., Allen, R.D. and Johnston, G.A. *Eur J Pharmacol* **233:** 169-172 (1993).
O'Hara, P.J., Sheppard, P.O., Thogersen, H., Venezia, D., Haldeman, B.A., McGrane, V., Houamed, K.M., Thomsen, C., Gilbert, T.L. and Mulvihill, E.R. *Neuron* **11**: 41-52 (1993)
Raiteri, M., Bonanno, G., Gemignani, A., Pende, M., Vellebuona, F.and Lanza, *M. Adv. Biochem. Psychopharmacol.* **47**: 205-216 (1992)
Romano, C., Yang, W-L. and O'Malley, K.L. *J*. *Biol.Chem.* **271**: 28612-28616 (1996).
Sambrook, J., Fritsch, E.F. and Maniatis, T. Molecular Cloning: a Laboratory Manual. 2^{nd} Edition. CSH Laboratory Press. (1989)
Sherman, F. *Methods Enzymol.* **194**: 3-21 (1991)
Smith, P. K., Krohn, R. I., Hermanson, G. T., Mallia, A. K., Gartner, F. H., Provenzano, M. D., Fujimoto, E. K., Goeke, N. M., Olson, B. J. and Klenk, D. C. *Anal. Biochem.* **150**: 76-85 (1985)
Takahashi, K, Tsuchida, K., Tanabe, Y., Masu, M. and Nakanishi, S. J. *Biol. Chem.* **268**: 19341-19345 (1993)
Uezono, Y., Bradley, J., Min, C., McCarty, N.A., Quick, M., Riordan, J.R., Chavkin, C., Zinn, K., Lester, A. and Davidson, N. *Receptors and Channels* **1***:* 233-241 (1993)
Wotta, D.R., Birnbaum, A.K., Wilcox, G.L., Elde, R. and Law, P.Y. *Brain Res. Mol. Brain Res.* **44:** 55-65 (1997)

## Claims

1. A GABA_{B} receptor comprising a heterodimer between a GABA_{B}-R1 receptor protein and a GABA_{B}-R2 receptor protein, wherein the GABA_{B}-R2 receptor protein has the amino acid sequence provided in Fig. 1B or an amino acid sequence that has at least 90 % homology to the amino acid sequence provided in Fig. 1B.

2. The GABA_{B}-receptor according to claim I, wherein the GABA_{B}-R1 receptor is a GABA_{B}-R1 receptor protein, a GABA_{B}-R1b receptor protein or a GABA_{B}-R1c receptor protein.

3. An isolated GABA_{B}-R2 receptor protein having the amino acid sequence provided in Fig. 1B.

4. A nucleotide sequence encoding a GABA_{B}-R2 receptor protein according to claim 3, or a nucleotide sequence which is complementary thereto over the full length.

5. A nucleotide sequence according to claim 4, having the nucleotide sequence shown in Fig. 1A or a nucleotide sequence which is complementary thereto over the full length.

6. A nucleotide sequence according to claim 4 or 5, which is a cDNA sequence.

7. An expression vector comprising a nucleotide sequence according to any one of claims 4 to 6, which is capable of expressing a GABA_{B}-R2 receptor protein.

8. An expression vector comprising a nucleotide sequence encoding a GABA_{B-}R1 receptor and a nucleotide sequence encoding a GABA_{B}-R2 receptor, said vector being capable of expressing both a GABA_{B}-R1 receptor protein and a GABA_{B}-R2 receptor protein, wherein the GABA_{B}-R2 receptor protein has the amino acid sequence provided in Fig. 1B or an amino acid sequence having at least 90 % homology to the amino acid sequence provided in Fig. 1B.

9. A vector according to claim 8, wherein the GABA_{B}-R1 receptor protein is a GABA_{B}-R1a receptor protein, a GABA_{B}-R1b receptor protein or a GABA_{B}-R1c receptor protein.

10. A stable cell line comprising a vector according to any one of claims 7 to 9.

11. A stable cell line modified to express both GABA_{B}-R1 and GABA_{B}-R2 receptor proteins, wherein the GABA_{B}-R2 receptor protein has the amino acid sequence provided in Fig. 1B or an amino acid sequence having at least 90 % homology to the amino acid sequence provided in Fig. 1B.

12. A stable cell line according to claim 10 or 11, which is a modified HEK293T cell line.

13. A GABA_{B} receptor comprising a heterodimer between a GABA_{B}-R1 receptor protein and a GABA_{B}-R2 receptor protein produced by a stable cell line according to any one of claims 10 to 12.

14. A GABA_{B}-R2 receptor protein produced by a stable cell line according to any one of claims 10 to 12.

15. An antibody specific for a GABA_{B} receptor according to claim 1 or 2.

16. An antibody specific for a GABA_{B}-R2 receptor protein according to claim 3.

17. A method for identification of a compound which exhibits GABA_{B} receptor modulating activity, which method comprises contacting a GABA_{B} receptor according to claim 1 or 2 with a test compound and detecting modulating activity or inactivity.

18. A method of producing a GABA_{B} receptor comprising introducing into an appropriate cell line a suitable vector or vectors comprising nucleotide sequences encoding GABA_{B}-R1 and GABA_{B}-R2 receptor proteins, under conditions suitable for obtaining expression of the receptor proteins or variants, wherein the GABA_{B}-R2 receptor protein has the amino acid sequence provided in Fig. 1B or an amino acid sequence having at least 90 % homology to the amino acid sequence provided in Fig. 1B.

## Patentansprüche

1. GABA_{B}-Rezeptor, umfassend ein Heterodimer zwischen einem GABA_{B}-R1-Rezeptorprotein und einem GABA_{B}-R2-Rezeptorprotein, wobei das GABA_{B}-R2-Rezeptorprotein die in Figur 1 B dargestellte Aminosäuresequenz oder eine Aminosäuresequenz aufweist, die mindestens 90% Homologie zu der in Figur 1 B dargestellten Aminosäuresequenz besitzt.

2. GABA_{B}-Rezeptor gemäß Anspruch 1, wobei der GABA_{B}-R1-Rezeptor ein GABA_{B}-R1-Rezeptorprotein, ein GABA_{B}-R1b-Rezeptorprotein oder ein GABA_{B}-R1c-Rezeptorprotein ist.

3. Isoliertes GABA_{B}-R2-Rezeptorprotein, das die in Figur 1 B dargestellte Aminosäuresequenz aufweist.

4. Nucleotidsequenz, die ein GABA_{B}-R2-Rezeptorprotein gemäß Anspruch 3 codiert, oder eine Nucleotidsequenz, die hierzu über die volle Länge komplementär ist.

5. Nucleotidsequenz gemäß Anspruch 4, die die in Figur 1 A dargestellte Nucleotidsequenz aufweist, oder eine Nucleotidsequenz, die hierzu über die volle Länge komplementär ist.

6. Nucleotidsequenz gemäß Anspruch 4 oder 5, die eine cDNA-Sequenz ist.

7. Expressionsvektor, umfassend eine Nucleotidsequenz gemäß einem der Ansprüche 4 bis 6, der zur Expression eines GABA_{B}-R2-Rezeptorproteins fähig ist.

8. Expressionsvektor, umfassend eine einen GABA_{B}-R1-Rezeptor codierende Nucleotidsequenz und eine einen GABA_{B}-R2-Rezeptor codierende Nucleotidsequenz, wobei der Vektor fähig ist, sowohl ein GABA_{B}-R1-Rezeptorprotein als auch ein GABA_{B}-R2-Rezeptorprotein zu exprimieren, wobei das GABA_{B}-R2-Rezeptorprotein die in Figur 1 B dargestellte Aminosäuresequenz oder eine Aminosäuresequenz aufweist, die mindestens 90% Homologie zu der in Figur 1 B dargestellten Aminosäuresequenz hat.

9. Vektor gemäß Anspruch 8, wobei das GABA_{B}-R1-Rezeptorprotein ein GABA_{B}-R1a-Rezeptorprotein, ein GABA_{B}-R1b-Rezeptorprotein oder ein GABA_{B}-R1c-Rezeptorprotein ist.

10. Stabile Zelllinie, umfassend einen Vektor gemäß einem der Ansprüche 7 bis 9.

11. Stabile Zelllinie, die so modifiziert ist, dass sie sowohl GABA_{B}-R1- als auch GABA_{B}-R2-Rezeptorproteine exprimiert, wobei das GABA_{B}-R2-Rezeptorprotein die in Figur 1 B dargestellte Aminosäuresequenz oder eine Aminosäuresequenz aufweist, die mindestens 90% Homologie zu der in Figur 1 B dargestellten Aminosäuresequenz hat.

12. Stabile Zelllinie gemäß Anspruch 10 oder 11, die eine modifizierte HEK293T-Zelllinie ist.

13. GABA_{B}-Rezeptor, umfassend ein Heterodimer zwischen einem GABA_{B}-R1-Rezeptorprotein und einem GABA_{B}-R2-Rezeptorprotein, produziert von einer stabilen Zelllinie gemäß einem der Ansprüche 10 bis 12.

14. GABA_{B}-R2-Rezeptorprotein, das von einer stabilen Zelllinie gemäß einem der Ansprüche 10 bis 12 produziert wird.

15. Antikörper, der für einen GABA_{B}-Rezeptor gemäß Anspruch 1 oder 2 spezifisch ist.

16. Antikörper, der für ein GABA_{B}-R2-Rezeptorprotein gemäß Anspruch 3 spezifisch ist.

17. Verfahren zur Identifizierung einer Verbindung, die GABA_{B-}Rezeptormodulationsaktivität zeigt, wobei das Verfahren das Inkontaktbringen eines GABA_{B}-Rezeptors gemäß Anspruch 1 oder 2 mit einer Testverbindung sowie den Nachweis der Modulationsaktivität oder des Fehlens einer solchen Aktivität umfasst.

18. Verfahren zur Herstellung eines GABA_{B}-Rezeptors, umfassend das Einbringen eines geeigneten Vektors oder von geeigneten Vektoren, die GABA_{B}-R1- und GABA_{B}-R2-Rezeptorproteine codierende Nucleotidsequenzen umfassen, in eine geeignete Zelllinie unter Bedingungen, die geeignet sind, die Expression des Rezeptorproteins oder von Varianten herbeizuführen, wobei das GABA_{B}-R2-Rezeptorprotein die in Figur 1 B dargestellte Aminosäuresequenz oder eine Aminosäuresequenz aufweist, die mindestens 90% Homologie zu der in Figur 1 B dargestellten Aminosäuresequenz hat.

## Revendications

1. Récepteur GABA_{B} comprenant un hétérodimère entre une protéine récepteur GABA_{B}-R1 et une protéine récepteur GABA_{B}-R2, dans lequel la protéine récepteur GABA_{B}-R2 a la séquence d'aminoacides indiquée sur la figure 1B ou une séquence d'aminoacides qui présente une homologie d'au moins 90 % avec la séquence d'aminoacides indiquée sur la figure 1B.

2. Récepteur GABA_{B} suivant la revendication 1, dans lequel le récepteur GABA_{B}-R1 est une protéine récepteur GABA_{B}-R1, une protéine récepteur GABA_{B}-R1b ou une protéine récepteur GABA_{B}-R1c.

3. Protéine récepteur GABA_{B}-R2 isolée ayant la séquence d'aminoacides indiquée sur la figure 1B.

4. Séquence de nucléotides codant pour une protéine récepteur GABA_{B}-R2 suivant la revendication 3, ou une séquence de nucléotides qui est complémentaire de celle-ci sur la longueur totale.

5. Séquence de nucléotides suivant la revendication 4, ayant la séquence de nucléotides représentée sur la figure 1A ou une séquence de nucléotides qui est complémentaire de celle-ci sur la longueur totale.

6. Séquence de nucléotides suivant la revendication 4 ou 5, qui est une séquence d'ADNc.

7. Vecteur d'expression comprenant une séquence de nucléotides suivant l'une quelconque des revendications 4 à 6, qui est capable d'exprimer une protéine récepteur GABA_{B}-R2.

8. Vecteur d'expression comprenant une séquence de nucléotides codant pour un récepteur GABA_{B}-R1, et une séquence de nucléotides codant pour un récepteur GABA_{B}-R2, ledit vecteur étant capable d'exprimer à la fois une protéine récepteur GABA_{B}-R1 et une protéine récepteur GABA_{B}-R2, dans lequel la protéine récepteur GABA_{B}-R2 a la séquence d'aminoacides indiquée sur la figure 1B ou une séquence d'aminoacides présentant une homologie d'au moins 90 % avec la séquence d'aminoacides indiquée sur la figure 1B.

9. Vecteur suivant la revendication 8, dans lequel la protéine récepteur GABA_{B}-R1 est une protéine récepteur GABA_{B}-R1a, une protéine récepteur GABA_{B}-R1b ou une protéine récepteur GABA_{B}-R1c.

10. Lignée cellulaire stable comprenant un vecteur suivant l'une quelconque des revendications 7 à 9.

11. Lignée cellulaire stable modifiée pour exprimer les deux protéines récepteurs GABA_{B}-R1 et GABA_{B}-R2, dans laquelle la protéine récepteur GABA_{B}-R2 a la séquence d'aminoacides indiquée sur la figure 1B ou une séquence d'aminoacides présentant une homologie d'au moins 90 % avec la séquence d'aminoacides indiquée sur la figure 1B.

12. Lignée cellulaire stable suivant la revendication 10 ou 11, qui est une lignée cellulaire HEK293T modifiée.

13. Récepteur GABA_{B} comprenant un hétérodimère entre une protéine récepteur GABA_{B}-R1 et une protéine récepteur GABA_{B}-R2 produit par une lignée cellulaire stable suivant l'une quelconque des revendications 10 à 12.

14. Protéine récepteur GABA_{B}-R2 produite par une lignée cellulaire stable suivant l'une quelconque des revendications 10 à 12.

15. Anticorps spécifique d'un récepteur GABA_{B} suivant la revendication 1 ou 2.

16. Anticorps spécifique d'une protéine récepteur GABA_{B}-R2 suivant la revendication 3.

17. Méthode pour identifier un composé qui présente une activité de modulation du récepteur GABA_{B}, méthode qui comprend la mise en contact d'un récepteur GABA_{B} suivant la revendication 1 ou 2 avec un composé d'essai et la détection de la modulation de l'activité ou de l'inactivité.

18. Procédé pour la production d'un récepteur GABA_{B}, comprenant l'introduction dans une lignée cellulaire appropriée d'un ou plusieurs vecteurs convenables comprenant des séquences de nucléotides codant pour les protéines récepteurs GABA_{B}-R1 et GABA_{B}-R2, dans des conditions convenables pour parvenir à l'expression des protéines récepteurs ou de leurs variants, dans lequel la protéine récepteur GABA_{B}-R2 a la séquence d'aminoacides indiquée sur la figure 1B ou une séquence d'aminoacides présentant une homologie d'au moins 90 % avec la séquence d'aminoacides indiquée sur la figure 1B.


### SEQUENCE LISTING

<110> Glaxo Group Limited
   Barnes, Ashley A
   Wise, Alan
   Marshall, Fiona H
   Fraser, Neil J
   White, Julia HM
   Foord, Steven M
<120> Novel Receptor
<130> PG3558
<140> PCT/GB99/02918
   <141> 1999-09-03
<150> GB 9819420.2
   <151> 1998-09-07
<150> US 60/103,670
   <151> 1998-10-09
<160> 34
<170> PatentIn Ver. 2.1
<210> 1
   <211> 2826
   <212> DNA
   <213> Homo sapiens
<400> 1
   atggcttccc cgcggagctc cgggcagccc gggccgccgc cgccgccgcc accgccgccc 60
   gcgcgcctgc tactgctact gctgctgccg ctgctgctgc ctctggcgcc cggggcctgg 120
   ggctgggcgc ggggcgcccc ccggccgccg cccagcagcc cgccgctctc catcatgggc 180
   ctcatgccgc tcaccaagga ggtggccaag ggcagcatcg ggcgcggtgt gctccccgcc 240
   gtggaactgg ccatcgagca gatccgcaac gagtcactcc tgcgccccta cttcctcgac 300
   ctgcggctct atgacacgga gtgcgacaac gcaaaagggt tgaaagcctt ctacgatgca 360
   ataaaatacg ggcctaacca cttgatggtg tttggaggcg tctgtccatc cgtcacatcc 420
   atcattgcag agtccctcca aggctggaat ctggtgcagc tttcttttgc tgcaaccacg 480
   cctgttctag ccgataagaa aaaataccct tatttctttc ggaccgtccc atcagacaat 540
   gcggtgaatc cagccattct gaagttgctc aagcactacc agtggaagcg cgtgggcacg 600
   ctgacgcaag acgttcagag gttctctgag gtgcggaatg acctgactgg agttctgtat 660
   ggcgaggaca ttgagatttc agacaccgag agcttctcca acgatccctg taccagtgtc 720
   aaaaagctga aggggaatga tgtgcggatc atccttggcc agtttgacca gaatatggca 780
   gcaaaagtgt tctgttgtgc atacgaggag aacatgtatg gtagtaaata tcagtggatc 840
   attccgggct ggtacgagcc ttcttggtgg gagcaggtgc acacggaagc caactcatcc 900
   cgctgcctcc ggaagaatct gcttgctgcc atggagggct acattggcgt ggatttcgag 960
   cccctgagct ccaagcagat caagaccatc tcaggaaaga ctccacagca gtatgagaga 1020
   gagtacaaca acaagcggtc aggcgtgggg cccagcaagt tccacgggta cgcctacgat 1080
   ggcatctggg tcatcgccaa gacactgcag agggccatgg agacactgca tgccagcagc 1140
   cggcaccagc ggatccagga cttcaactac acggaccaca cgctgggcag gatcatcctc 1200
   aatgccatga acgagaccaa cttcttcggg gtcacgggtc aagttgtatt ccggaatggg 1260
   gagagaatgg ggaccattaa atttactcaa tttcaagaca gcagggaggt gaaggtggga 1320
   gagtacaacg ctgtggccga cacactggag atcatcaatg acaccatcag gttccaagga 1380
   tccgaaccac caaaagacaa gaccatcatc ctggagcagc tgcggaagat ctccctacct 1440
   ctctacagca tcctctctgc cctcaccatc ctcgggatga tcatggccag tgcttttctc 1500
   ttcttcaaca tcaagaaccg gaatcagaag ctcataaaga tgtcgagtcc atacatgaac 1560
   aaccttatca tccttggagg gatgctctcc tatgcttcca tatttctctt tggccttgat 1620
   ggatcctttg tctctgaaaa gacctttgaa acactttgca ccgtcaggac ctggattctc 1680
   accgtgggct acacgaccgc ttttggggcc atgtttgcaa agacctggag agtccacgcc 1740
   atcttcaaaa atgtgaaaat gaagaagaag atcatcaagg accagaaact gcttgtgatc 1800
   gtggggggca tgctgctgat cgacctgtgt atcctgatct gctggcaggc tgtggacccc 1860
   ctgcgaagga cagtggagaa gtacagcatg gagccggacc cagcaggacg ggatatctcc 1920
   atccgccctc tcctggagca ctgtgagaac acccatatga ccatctggct tggcatcgtc 1980
   tatgcctaca agggacttct catgttgttc ggttgtttct tagcttggga gacccgcaac 2040
   gtcagcatcc ccgcactcaa cgacagcaag tacatcggga tgagtgtcta caacgtgggg 2100
   atcatgtgca tcatcggggc cgctgtctcc ttcctgaccc gggaccagcc caatgtgcag 2160
   ttctgcatcg tggctctggt catcatcttc tgcagcacca tcaccctctg cctggtattc 2220
   gtgccgaagc tcatcaccct gagaacaaac ccagatgcag caacgcagaa caggcgattc 2280
   cagttcactc agaatcagaa gaaagaagat tctaaaacgt ccacctcggt caccagtgtg 2340
   aaccaagcca gcacatcccg cctggagggc ctacagtcag aaaaccatcg cctgcgaatg 2400
   aagatcacag agctggataa agacttggaa gaggtcacca tgcagctgca ggacacacca 2460
   gaaaagacca cctacattaa acagaaccac taccaagagc tcaatgacat cctcaacctg 2520
   ggaaacttca ctgagagcac agatggagga aaggccattt taaaaaatca cctcgatcaa 2580
   aatccccagc tacagtggaa cacaacagag ccctctcgaa catgcaaaga tcctatagaa 2640
   gatataaact ctccagaaca catccagcgt cggctgtccc tccagctccc catcctccac 2700
   cacgcctacc tcccatccat cggaggcgtg gacgccagct gtgtcagccc ctgcgtcagc 2760
   cccaccgcca gcccccgcca cagacatgtg ccaccctcct tccgagtcat ggtctcgggc 2820
   ctgtaa 2826
<210> 2
   <211> 941
   <212> PRT
   <213> Homo sapiens
<400> 2
<210> 3
   <211> 960
   <212> PRT
   <213> Homo sapiens
<400> 3
   Met Leu Leu Leu Leu Leu Ala Pro Leu Phe Leu Arg Pro Pro Gly Ala
   1               5                   10                  15
   Gly Gly Ala Gln Thr Pro Asn Ala Thr Ser Glu Gly Cys Gln Ile Ile
               20                  25                  30
   His Pro Pro Trp Glu Gly Gly Ile Arg Tyr Arg Gly Leu Thr Arg Asp
           35                  40                  45
   Gln Val Lys Ala Ile Asn Phe Leu Pro Val Asp Tyr Glu Ile Glu Tyr
       50                  55                  60
   Val Cys Arg Gly Glu Arg Glu Val Val Gly Pro Lys Val Arg Lys Cys
   65                  70                  75                  80
   Leu Ala Asn Gly Ser Trp Thr Asp Met Asp Thr Pro Ser Arg Cys Val
                   85                  90                  95
   Arg Ile Cys Ser Lys Ser Tyr Leu Thr Leu Glu Asn Gly Lys Val Phe
               100                 105                 110
   Leu Thr Gly Gly Asp Leu Pro Ala Leu Asp Gly Ala Arg Val Asp Phe
           115                 120                 125
   Arg Cys Asp Pro Asp Phe His Leu Val Gly Ser Ser Arg Ser Ile Cys
       130                 135                 140
   Ser Gln Gly Gln Trp Ser Thr Pro Lys Pro His Cys Gln Val Asn Arg
   145                 150                 155                 160
   Thr Pro His Ser Glu Arg Arg Ala Val Tyr Ile Gly Ala Leu Phe Pro
                   165                 170                 175
   Met Ser Gly Gly Trp Pro Gly Gly Gln Ala Cys Gln Pro Ala Val Glu
   180 185 190
   Met Ala Leu Glu Asp Val Asn Ser Arg Arg Asp Ile Leu Pro Asp Tyr
   195 200 205
   Glu Leu Lys Leu Ile His His Asp Ser Lys Cys Asp Pro Gly Gln Ala
   210 215 220
   Thr Lys Tyr Leu Tyr Glu Leu Leu Tyr Asn Asp Pro Ile Lys Ile Ile
   225 230 235 240
   Leu Met Pro Gly Cys Ser Ser Val Ser Thr Leu Val Ala Glu Ala Ala
   245 250 255
   Arg Met Trp Asn Leu Ile Val Leu Ser Tyr Gly Ser Ser Ser Pro Ala
   260 265 270
   Leu Ser Asn Arg Gln Arg Phe Pro Thr Phe Phe Arg Thr His Pro Ser
   275 280 285
   Ala Thr Leu His Asn Pro Thr Arg Val Lys Leu Phe Glu Lys Trp Gly
   290 295 300
   Trp Lys Lys Ile Ala Thr Ile Gln Gln Thr Thr Glu Val Phe Thr Ser
   305 310 315 320
   Thr Leu Asp Asp Leu Glu Glu Arg Val Lys Glu Ala Gly Ile Glu Ile
   325 330 335
   Thr Phe Arg Gln Ser Phe Phe Ser Asp Pro Ala Val Pro Val Lys Asn
   340 345 350
   Leu Lys Arg Gln Asp Ala Arg Ile Ile Val Gly Leu Phe Tyr Glu Thr
   355 360 365
   Glu Ala Arg Lys Val Phe Cys Glu Val Tyr Lys Glu Arg Leu Phe Gly
   370 375 380
   Lys Lys Tyr Val Trp Phe Leu Ile Gly Trp Tyr Ala Asp Asn Trp Phe
   385 390 395 400
   Lys Ile Tyr Asp Pro Ser Ile Asn Cys Thr Val Asp Glu Met Thr Glu
   405 410 415
   Ala Val Glu Gly His Ile Thr Thr Glu Ile Val Met Leu Asn Pro Ala
   420 425 430
   Asn Thr Arg Ser Ile Ser Asn Met Thr Ser Gln Glu Phe Val Glu Lys
   435 440 445
   Leu Thr Lys Arg Leu Lys Arg His Pro Glu Glu Thr Gly Gly Phe Gln
   450 455 460
   Glu Ala Pro Leu Ala Tyr Asp Ala Ile Trp Ala Leu Ala Leu Ala Leu
   465 470 475 480
   Asn Lys Thr Ser Gly Gly Gly Gly Arg Ser Gly Val Arg Leu Glu Asp
   485 490 495
   Phe Asn Tyr Asn Asn Gln Thr Ile Thr Asp Gln Ile Tyr Arg Ala Met
   500 505 510
   Asn Ser Ser Ser Phe Glu Gly Val Ser Gly His Val Val Phe Asp Ala
   515 520 525
   Ser Gly Ser Arg Met Ala Trp Thr Leu Ile Glu Gln Pro Gln Gly Gly
   530 535 540
   Ser Tyr Lys Lys Ile Gly Tyr Tyr Asp Ser Thr Lys Asp Asp Leu Ser
   545 550 555 560
   Trp Ser Lys Thr Asp Lys Trp Ile Gly Gly Ser Pro Pro Ala Asp Gln
   565 570 575
   Thr Leu Val Ile Lys Thr Phe Arg Phe Leu Ser Gln Lys Leu Phe Ile
   580 585 590
   Ser Val Ser Val Leu Ser Ser Leu Gly Ile Val Leu Ala Val Val Cys
   595 600 605
   Leu Ser Phe Asn Ile Tyr Asn Ser His Val Arg Tyr Ile Gln Asn Ser
   610 615 620
   Gln Pro Asn Leu Asn Asn Leu Thr Ala Val Gly Cys Ser Leu Ala Leu
   625 630 635 640
   Ala Ala Val Phe Pro Leu Gly Leu Asp Gly Tyr His Ile Gly Arg Asn
   645 650 655
   Gln Phe Pro Phe Val Cys Gln Ala Arg Leu Trp Leu Leu Gly Leu Gly
   660 665 670
   Phe Ser Leu Gly Tyr Gly Ser Met Phe Thr Lys Ile Trp Trp Val His
   675 680 685
   Thr Gly Phe Thr Lys Lys Glu Glu Lys Lys Glu Trp Arg Lys Thr Leu
   690 695 700
   Glu Pro Trp Lys Leu Tyr Ala Thr Val Gly Leu Leu Val Gly Met Asp
   705 710 715 720
   Val Leu Thr Leu Ala Ile Trp Gln Ile Val Asp Pro Leu His Arg Thr
   725 730 735
   Ile Glu Thr Phe Ala Lys Glu Glu Pro Lys Glu Asp Ile Asp Val Ser
   740 745 750
   Ile Leu Pro Gln Leu Glu His Cys Ser Ser Arg Lys Met Asn Thr Trp
   755 760 765
   Leu Gly Ile Phe Tyr Gly Tyr Lys Gly Leu Leu Leu Leu Leu Gly Ile
   770 775 780
   Phe Leu Ala Tyr Glu Thr Lys Ser Val Ser Thr Glu Lys Ile Asn Asp
   785 790 795 800
   His Arg Ala Val Gly Met Ala Ile Tyr Asn Val Ala Val Leu Cys Leu
   805 810 815
   Ile Thr Ala Pro Val Thr Met Ile Leu Ser Ser Gln Gln Asp Ala Ala
   820 825 830
   Phe Ala Phe Ala Ser Leu Ala Ile Val Phe Ser Ser Tyr Ile Thr Leu
   835 840 845
   Val Val Leu Phe Val Pro Lys Met Arg Arg Leu Ile Thr Arg Gly Glu
   850 855 860
   Trp Gln Ser Glu Ala Gln Asp Thr Met Lys Thr Gly Ser Ser Thr Asn
   865 870 875 880
   Asn Asn Glu Glu Glu Lys Ser Arg Leu Leu Glu Lys Glu Asn Arg Glu
   885 890 895
   Leu Glu Lys Ile Ile Ala Glu Lys Glu Glu Arg Val Ser Glu Leu Arg
   900 905 910
   His Gln Leu Gln Ser Arg Gln Gln Leu Arg Ser Arg Arg His Pro Pro
   915 920 925
   Thr Pro Pro Glu Pro Ser Gly Gly Leu Pro Arg Gly Pro Pro Glu Pro
   930 935 940
   Pro Asp Arg Leu Ser Cys Asp Gly Ser Arg Val His Leu Leu Tyr Lys
   945 950 955 960
<210> 4
   <211> 844
   <212> PRT
   <213> Homo sapiens
<400> 4
   Met Gly Pro Gly Ala Pro Phe Ala Arg Val Gly Trp Pro Leu Pro Leu
   1 5 10 15
   Leu Val Val Met Ala Ala Gly Val Ala Pro Val Trp Ala Ser His Ser
   20 25 30
   Pro His Leu Pro Arg Pro His Ser Arg Val Pro Pro His Pro Ser Ser
   35 40 45
   Glu Arg Arg Ala Val Tyr Ile Gly Ala Leu Phe Pro Met Ser Gly Gly
   50 55 60
   Trp Pro Gly Gly Gln Ala Cys Gln Pro Ala Val Glu Met Ala Leu Glu
   65 70 75 80
   Asp Val Asn Ser Arg Arg Asp Ile Leu Pro Asp Tyr Glu Leu Lys Leu
   85 90 95
   Ile His His Asp Ser Lys Cys Asp Pro Gly Gln Ala Thr Lys Tyr Leu
   100 105 110
   Tyr Glu Leu Leu Tyr Asn Asp Pro Ile Lys Ile Ile Leu Met Pro Gly
   115 120 125
   Cys Ser Ser Val Ser Thr Leu Val Ala Glu Ala Ala Arg Met Trp Asn
   130 135 140
   Leu Ile Val Leu Ser Tyr Gly Ser Ser Ser Pro Ala Leu Ser Asn Arg
   145 150 155 160
   Gln Arg Phe Pro Thr Phe Phe Arg Thr His Pro Ser Ala Thr Leu His
   165 170 175
   Asn Pro Thr Arg Val Lys Leu Phe Glu Lys Trp Gly Trp Lys Lys Ile
   180 185 190
   Ala Thr Ile Gln Gln Thr Thr Glu Val Phe Thr Ser Thr Leu Asp Asp
   195 200 205
   Leu Glu Glu Arg Val Lys Glu Ala Gly Ile Glu Ile Thr Phe Arg Gln
   210 215 220
   Ser Phe Phe Ser Asp Pro Ala Val Pro Val Lys Asn Leu Lys Arg Gln
   225 230 235 240
   Asp Ala Arg Ile Ile Val Gly Leu Phe Tyr Glu Thr Glu Ala Arg Lys
   245 250 255
   Val Phe Cys Glu Val Tyr Lys Glu Arg Leu Phe Gly Lys Lys Tyr Val
   260 265 270
   Trp Phe Leu Ile Gly Trp Tyr Ala Asp Asn Trp Phe Lys Ile Tyr Asp
   275 280 285
   Pro Ser Ile Asn Cys Thr Val Asp Glu Met Thr Glu Ala Val Glu Gly
   290 295 300
   His Ile Thr Thr Glu Ile Val Met Leu Asn Pro Ala Asn Thr Arg Ser
   305 310 315 320
   Ile Ser Asn Met Thr Ser Gln Glu Phe Val Glu Lys Leu Thr Lys Arg
   325 330 335
   Leu Lys Arg His Pro Glu Glu Thr Gly Gly Phe Gln Glu Ala Pro Leu
   340 345 350
   Ala Tyr Asp Ala Ile Trp Ala Leu Ala Leu Ala Leu Asn Lys Thr Ser
   355 360 365
   Gly Gly Gly Gly Arg Ser Gly Val Arg Leu Glu Asp Phe Asn Tyr Asn
   370 375 380
   Asn Gln Thr Ile Thr Asp Gln Ile Tyr Arg Ala Met Asn Ser Ser Ser
   385 390 395 400
   Phe Glu Gly Val Ser Gly His Val Val Phe Asp Ala Ser Gly Ser Arg
   405 410 415
   Met Ala Trp Thr Leu Ile Glu Gln Pro Gln Gly Gly Ser Tyr Lys Lys
   420 425 430
   Ile Gly Tyr Tyr Asp Ser Thr Lys Asp Asp Leu Ser Trp Ser Lys Thr
   435 440 445
   Asp Lys Trp Ile Gly Gly Ser Pro Pro Ala Asp Gln Thr Leu Val Ile
   450 455 460
   Lys Thr Phe Arg Phe Leu Ser Gln Lys Leu Phe Ile Ser Val Ser Val
   465 470 475 480
   Leu Ser Ser Leu Gly Ile Val Leu Ala Val Val Cys Leu Ser Phe Asn
   485 490 495
   Ile Tyr Asn Ser His Val Arg Tyr Ile Gln Asn Ser Gln Pro Asn Leu
   500 505 510
   Asn Asn Leu Thr Ala Val Gly Cys Ser Leu Ala Leu Ala Ala Val Phe
   515 520 525
   Pro Leu Gly Leu Asp Gly Tyr His Ile Gly Arg Asn Gln Phe Pro Phe
   530 535 540
   Val Cys Gln Ala Arg Leu Trp Leu Leu Gly Leu Gly Phe Ser Leu Gly
   545 550 555 560
   Tyr Gly Ser Met Phe Thr Lys Ile Trp Trp Val His Thr Gly Phe Thr
   565 570 575
   Lys Lys Glu Glu Lys Lys Glu Trp Arg Lys Thr Leu Glu Pro Trp Lys
   580 585 590
   Leu Tyr Ala Thr Val Gly Leu Leu Val Gly Met Asp Val Leu Thr Leu
   595 600 605
   Ala Ile Trp Gln Ile Val Asp Pro Leu His Arg Thr Ile Glu Thr Phe
   610 615 620
   Ala Lys Glu Glu Pro Lys Glu Asp Ile Asp Val Ser Ile Leu Pro Gln
   625 630 635 640
   Leu Glu His Cys Ser Ser Arg Lys Met Asn Thr Trp Leu Gly Ile Phe
   645 650 655
   Tyr Gly Tyr Lys Gly Leu Leu Leu Leu Leu Gly Ile Phe Leu Ala Tyr
   660 665 670
   Glu Thr Lys Ser Val Ser Thr Glu Lys Ile Asn Asp His Arg Ala Val
   675 680 685
   Gly Met Ala Ile Tyr Asn Val Ala Val Leu Cys Leu Ile Thr Ala Pro
   690 695 700
   Val Thr Met Ile Leu Ser Ser Gln Gln Asp Ala Ala Phe Ala Phe Ala
   705 710 715 720
   Ser Leu Ala Ile Val Phe Ser Ser Tyr Ile Thr Leu Val Val Leu Phe
   725 730 735
   Val Pro Lys Met Arg Arg Leu Ile Thr Arg Gly Glu Trp Gln Ser Glu
   740 745 750
   Ala Gln Asp Thr Met Lys Thr Gly Ser Ser Thr Asn Asn Asn Glu Glu
   755 760 765
   Glu Lys Ser Arg Leu Leu Glu Lys Glu Asn Arg Glu Leu Glu Lys Ile
   770 775 780
   Ile Ala Glu Lys Glu Glu Arg Val Ser Glu Leu Arg His Gln Leu Gln
   785 790 795 800
   Ser Arg Gln Gln Leu Arg Ser Arg Arg His Pro Pro Thr Pro Pro Glu
   805 810 815
   Pro Ser Gly Gly Leu Pro Arg Gly Pro Pro Glu Pro Pro Asp Arg Leu
   820 825 830
   Ser Cys Asp Gly Ser Arg Val His Leu Leu Tyr Lys
   835 840
<210> 5
   <211> 898
   <212> PRT
   <213> Homo sapiens
<400> 5
   Met Leu Leu Leu Leu Leu Ala Pro Leu Phe Leu Arg Pro Pro Gly Ala
   1 5 10 15
   Gly Gly Ala Gln Thr Pro Asn Ala Thr Ser Glu Gly Cys Gln Ile Ile
   20 25 30
   His Pro Pro Trp Glu Gly Gly Ile Arg Tyr Arg Gly Leu Thr Arg Asp
   35 40 45
   Gln Val Lys Ala Ile Asn Phe Leu Pro Val Asp Tyr Glu Ile Glu Tyr
   50 55 60
   Val Cys Arg Gly Glu Arg Glu Val Val Gly Pro Lys Val Arg Lys Cys
   65 70 75 80
   Leu Ala Asn Gly Ser Trp Thr Asp Met Asp Thr Pro Ser Arg Cys Val
   85 90 95
   Asn Arg Thr Pro His Ser Glu Arg Arg Ala Val Tyr Ile Gly Ala Leu
   100 105 110
   Phe Pro Met Ser Gly Gly Trp Pro Gly Gly Gln Ala Cys Gln Pro Ala
           115                 120                 125
   Val Glu Met Ala Leu Glu Asp Val Asn Ser Arg Arg Asp Ile Leu Pro
       130                 135                 140
   Asp Tyr Glu Leu Lys Leu Ile His His Asp Ser Lys Cys Asp Pro Gly
   145                 150                 155                 160
   Gln Ala Thr Lys Tyr Leu Tyr Glu Leu Leu Tyr Asn Asp Pro Ile Lys
                   165                 170                 175
   Ile Ile Leu Met Pro Gly Cys Ser Ser Val Ser Thr Leu Val Ala Glu
               180                 185                 190
   Ala Ala Arg Met Trp Asn Leu Ile Val Leu Ser Tyr Gly Ser Ser Ser
           195                 200                 205
   Pro Ala Leu Ser Asn Arg Gln Arg Phe Pro Thr Phe Phe Arg Thr His
       210                 215                 220
   Pro Ser Ala Thr Leu His Asn Pro Thr Arg Val Lys Leu Phe Glu Lys
   225                 230                 235                 240
   Trp Gly Trp Lys Lys Ile Ala Thr Ile Gln Gln Thr Thr Glu Val Phe
                   245                 250                 255
   Thr Ser Thr Leu Asp Asp Leu Glu Glu Arg Val Lys Glu Ala Gly Ile
               260                 265                 270
   Glu Ile Thr Phe Arg Gln Ser Phe Phe Ser Asp Pro Ala Val Pro Val
           275                 280                 285
   Lys Asn Leu Lys Arg Gln Asp Ala Arg Ile Ile Val Gly Leu Phe Tyr
       290                 295                 300
   Glu Thr Glu Ala Arg Lys Val Phe Cys Glu Val Tyr Lys Glu Arg Leu
   305                 310                 315                 320
   Phe Gly Lys Lys Tyr Val Trp Phe Leu Ile Gly Trp Tyr Ala Asp Asn
   325 330 335
   Trp Phe Lys Ile Tyr Asp Pro Ser Ile Asn Cys Thr Val Asp Glu Met
   340 345 350
   Thr Glu Ala Val Glu Gly His Ile Thr Thr Glu Ile Val Met Leu Asn
   355 360 365
   Pro Ala Asn Thr Arg Ser Ile Ser Asn Met Thr Ser Gln Glu Phe Val
   370 375 380
   Glu Lys Leu Thr Lys Arg Leu Lys Arg His Pro Glu Glu Thr Gly Gly
   385 390 395 400
   Phe Gln Glu Ala Pro Leu Ala Tyr Asp Ala Ile Trp Ala Leu Ala Leu
   405 410 415
   Ala Leu Asn Lys Thr Ser Gly Gly Gly Gly Arg Ser Gly Val Arg Leu
   420 425 430
   Glu Asp Phe Asn Tyr Asn Asn Gln Thr Ile Thr Asp Gln Ile Tyr Arg
   435 440 445
   Ala Met Asn Ser Ser Ser Phe Glu Gly Val Ser Gly His Val Val Phe
   450 455 460
   Asp Ala Ser Gly Ser Arg Met Ala Trp Thr Leu Ile Glu Gln Pro Gln
   465 470 475 480
   Gly Gly Ser Tyr Lys Lys Ile Gly Tyr Tyr Asp Ser Thr Lys Asp Asp
   485 490 495
   Leu Ser Trp Ser Lys Thr Asp Lys Trp Ile Gly Gly Ser Pro Pro Ala
   500 505 510
   Asp Gln Thr Leu Val Ile Lys Thr Phe Arg Phe Leu Ser Gln Lys Leu
   515 520 525
   Phe Ile Ser Val Ser Val Leu Ser Ser Leu Gly Ile Val Leu Ala Val
   530 535 540
   Val Cys Leu Ser Phe Asn Ile Tyr Asn Ser His Val Arg Tyr Ile Gln
   545 550 555 560
   Asn Ser Gln Pro Asn Leu Asn Asn Leu Thr Ala Val Gly Cys Ser Leu
   565 570 575
   Ala Leu Ala Ala Val Phe Pro Leu Gly Leu Asp Gly Tyr His Ile Gly
   580 585 590
   Arg Asn Gln Phe Pro Phe Val Cys Gln Ala Arg Leu Trp Leu Leu Gly
   595 600 605
   Leu Gly Phe Ser Leu Gly Tyr Gly Ser Met Phe Thr Lys Ile Trp Trp
   610 615 620
   Val His Thr Gly Phe Thr Lys Lys Glu Glu Lys Lys Glu Trp Arg Lys
   625 630 635 640
   Thr Leu Glu Pro Trp Lys Leu Tyr Ala Thr Val Gly Leu Leu Val Gly
   645 650 655
   Met Asp Val Leu Thr Leu Ala Ile Trp Gln Ile Val Asp Pro Leu His
   660 665 670
   Arg Thr Ile Glu Thr Phe Ala Lys Glu Glu Pro Lys Glu Asp Ile Asp
   675 680 685
   Val Ser Ile Leu Pro Gln Leu Glu His Cys Ser Ser Arg Lys Met Asn
   690 695 700
   Thr Trp Leu Gly Ile Phe Tyr Gly Tyr Lys Gly Leu Leu Leu Leu Leu
   705 710 715 720
   Gly Ile Phe Leu Ala Tyr Glu Thr Lys Ser Val Ser Thr Glu Lys Ile
   725 730 735
   Asn Asp His Arg Ala Val Gly Met Ala Ile Tyr Asn Val Ala Val Leu
   740 745 750
   Cys Leu Ile Thr Ala Pro Val Thr Met Ile Leu Ser Ser Gln Gln Asp
   755 760 765
   Ala Ala Phe Ala Phe Ala Ser Leu Ala Ile Val Phe Ser Ser Tyr Ile
   770 775 780
   Thr Leu Val Val Leu Phe Val Pro Lys Met Arg Arg Leu Ile Thr Arg
   785 790 795 800
   Gly Glu Trp Gln Ser Glu Ala Gln Asp Thr Met Lys Thr Gly Ser Ser
   805 810 815
   Thr Asn Asn Asn Glu Glu Glu Lys Ser Arg Leu Leu Glu Lys Glu Asn
   820 825 830
   Arg Glu Leu Glu Lys Ile Ile Ala Glu Lys Glu Glu Arg Val Ser Glu
   835 840 845
   Leu Arg His Gln Leu Gln Ser Arg Gln Gln Leu Arg Ser Arg Arg His
   850 855 860
   Pro Pro Thr Pro Pro Glu Pro Ser Gly Gly Leu Pro Arg Gly Pro Pro
   865 870 875 880
   Glu Pro Pro Asp Arg Leu Ser Cys Asp Gly Ser Arg Val His Leu Leu
   885 890 895
   Tyr Lys
<210> 6
   <211> 26
   <212> DNA
   <213> Artificial Sequence
<220>
   <223> Description of Artificial Sequence: Primer
<400> 6
   gcgactgctg tgggctgctt actggc 26
<210> 7
   <211> 30
   <212> DNA
   <213> Artificial Sequence
<220>
   <223> Description of Artificial Sequence: Primer
<400> 7
   gcgaattccc tgtcctccct caccctaccc 30
<210> 8
   <211> 24
   <212> DNA
   <213> Artificial Sequence
<220>
   <223> Description of Artificial Sequence: Primer
<400> 8
   ccgagctcaa gctcatccac cacg 24
<210> 9
   <211> 24
   <212> DNA
   <213> Artificial Sequence
<220>
   <223> Description of Artificial Sequence: Primer
<400> 9
   tcttcctcca ctccttcttt tctt 24
<210> 10
   <211> 27
   <212> DNA
   <213> Artificial Sequence
<220>
   <223> Description of Artificial Sequence: Primer
<400> 10
   tgagctggag ccataggaaa gcacaat 27
<210> 11
   <211> 26
   <212> DNA
   <213> Artificial Sequence
<220>
   <223> Description of Artificial Sequence: Primer
<400> 11
   gatcttgata gggtcgttgt agagca 26
<210> 12
   <211> 20
   <212> DNA
   <213> Artificial Sequence
<220>
   <223> Description of Artificial Sequence: Primer
<400> 12
   gctcctaacg ctccccaaca 20
<210> 13
   <211> 20
   <212> DNA
   <213> Artificial Sequence
<220>
   <223> Description of Artificial Sequence: Primer
<400> 13
   ggcctggatc acacttgctg 20
<210> 14
   <211> 19
   <212> DNA
   <213> Artificial Sequence
<220>
   <223> Description of Artificial Sequence: Primer
<400> 14
   cccaacgcca cctcagaag 19
<210> 15
   <211> 19
   <212> DNA
   <213> Artificial Sequence
<220>
   <223> Description of Artificial Sequence: Primer
<400> 15
   ccgctcatgg gaaacagtg 19
<210> 16
   <211> 27
   <212> DNA
   <213> Artificial Sequence
<220>
   <223> Description of Artificial Sequence: Primer
<400> 16
   ccccggcaca catactcaat ctcatag 27
<210> 17
   <211> 22
   <212> DNA
   <213> Artificial Sequence
<220>
   <223> Description of Artificial Sequence: Primer
<400> 17
   ccggtacctg atgccccctt cc 22
<210> 18
   <211> 22
   <212> DNA
   <213> Artificial Sequence
<220>
   <223> Description of Artificial Sequence: Primer
<400> 18
   cgagatgttg ctgctgctgc ta 22
<210> 19
   <211> 23
   <212> DNA
   <213> Artificial Sequence
<220>
   <223> Description of Artificial Sequence: Primer
<400> 19
   taggatccca ctccccccat ccc 23
<210> 20
   <211> 20
   <212> DNA
   <213> Artificial Sequence
<220>
   <223> Description of Artificial Sequence: Primer
<400> 20
   ccagcgtgga gacagagctg 20
<210> 21
   <211> 17
   <212> DNA
   <213> Artificial Sequence
<220>
   <223> Description of Artificial Sequence: Primer
<400> 21
   atggcttccc cgcggag 17
<210> 22
   <211> 18
   <212> DNA
   <213> Artificial Sequence
<220>
   <223> Description of Artificial Sequence: Primer
<400> 22
   gaacaggcgt ggttgcag 18
<210> 23
   <211> 48
   <212> DNA
   <213> Artificial Sequence
<220>
   <223> Description of Artificial Sequence: Linker
<400*> 23
   agcttctcga ggcttgggga tgggcacgag gagctcctgc tcggccgg 48
<210> 24
   <211> 48
   <212> DNA
   <213> Artificial Sequence
<220>
   <223> Description of Artificial Sequence: Linker
<400> 24
   aattccggcc gagctggtgc tcctcgtgcc catccccaag cctcgaga 48
<210> 25
   <211> 10
   <212> PRT
   <213> Homo sapiens
<400> 25
   Ala Trp Gly Trp Ala Arg Gly Ala Pro Arg
   1 5 10
<210> 26
   <211> 23
   <212> DNA
   <213> Artificial Sequence
<220>
   <223> Description of Artificial Sequence: Primer
<400> 26
   cgcctggagg acttcaacta caa 23
<210> 27
   <211> 23
   <212> DNA
   <213> Artificial Sequence
<220>
   <223> Description of Artificial Sequence: Primer
<400> 27
   tcctcccaat gtggtaacca tcg 23
<210> 28
   <211> 19
   <212> DNA
   <213> Artificial Sequence
<220>
   <223> Description of Artificial Sequence: Primer
<400> 28
   acaagaccat catcctgga 19
<210> 29
   <211> 21
   <212> DNA
   <213> Artificial Sequence
<220>
   <223> Description of Artificial Sequence: Primer
<400> 29
   gatcacaagc agtttctggt c 21
<210> 30
   <211> 39
   <212> DNA
   <213> Artificial Sequence
<220>
   <223> Description of Artificial Sequence: Primer
<400> 30
   gttgtcccca tggtgcccaa gatgcgcagg ctgatcacc 39
<210> 31
   <211> 43
   <212> DNA
   <213> Artificial Sequence
<220>
   <223> Description of Artificial Sequence: Primer
<400> 31
   gtcctgcggc cgcggatcct cacttataaa gcaaatgcac tcg 43
<210> 32
   <211> 47
   <212> DNA
   <213> Artificial Sequence
<220>
   <223> Description of Artificial Sequence: Primer
<400> 32
   ctctgcccca tggccgtgcc gaagctcatc accctgagaa caaaccc 47
<210> 33
   <211> 51
   <212> DNA
   <213> Artificial Sequence
<220>
   <223> Description of Artificial Sequence: Primer
<400> 33
   ggcccagggc ggccgcactt acaggcccga gaccatgact cggaaggagg g 51
<210> 34
   <211> 27
   <212> DNA
   <213> Artificial Sequence
<220>
   <223> Description of Artificial Sequence: Primer
<400> 34
   cagggatgtt taataccact acaatgg 27